# EUROPEAN PATENT APPLICATION

(11) **EP 2 716 631 A1**
(43) Date of publication of application: **09.04.2014**
(21) Application number: 12793540.1
(22) Date of filing: 25.05.2012
(51) Int. Cl.: C07D 209/52

(54) **METHOD FOR PRODUCING PROLINE COMPOUND**

(30) Priority: 03.06.2011 JP 2011125579; 14.12.2011 JP 2011273342
(71) Applicant: Sumitomo Chemical Co., Ltd, Chuo-ku, Tokyo 104-8260 (JP)
(72) Inventor: WANG, Weiqi, Osaka-shi Osaka 555-0021 (JP); SHIMASAKI, Yasuharu, Osaka-shi Osaka 555-0021 (JP); IKEMOTO, Tetsuya, Osaka-shi Osaka 555-0021 (JP)
(74) Representative: Nieuwenhuys, William Francis
(86) International application number: PCT/JP2012/064248
(87) International publication number: WO 2012/165607

(57) **Abstract**

A proline compound represented by formula (1), 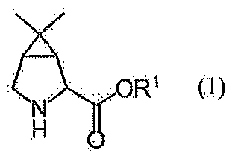 or a salt thereof, which is useful as an intermediate for the production of a medicinal or agrochemical agent or the like, can be produced by a method comprising the following steps A to D:
[Step A] :
a step of reacting a pyrrolidinone compound with a reducing agent, **(M^{s+})_{1/s}H₄₋ₘAI⁻(OR³)m**, in the presence of abase;
[Step B]:
a step of reacting a pyrrolidinol compound with a cyanating agent;
[Step C]:
a step of hydrolyzing or alcoholyzing the cyanopyrrolidine compound, and
[Step D]:
a step of substituting R² in the protected proline compound by a hydrogen atom.

## Description

### TECHNICAL FIELD

The present invention relates to a method for producing a proline compound and the like.

### BACKGROUND ART

A proline compound represented by formula (1) 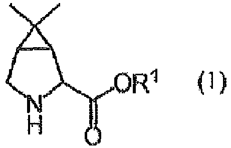 wherein R¹ represents an optionally substituted aliphatic hydrocarbon group or a hydrogen atom,
or a salt thereof is useful as an intermediate for the production of a medicinal or agrochemical agent or the like. For example, methods for producing a hydrochloride salt of a proline compound represented by formula (1a) 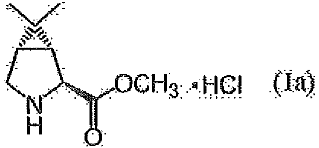 are reported in US2005/0059648, US2009/0240063,
US2010/0145069 and US2010/0063300. The method described in US2005/0059648, US2009/0240063 and US2010/0145069 is a method for producing a proline compound from a succinic anhydride derivative, but has problems that there are a large number of steps, and the yield is not always sufficient. In addition, the method described in US2010/0063300 is a method for producing a hydrochloride salt of a proline compound from a pyrrolidine derivative, using an enzyme such as oxidase.

The present invention provides a method for producing a proline compound represented by formula (1) or a salt thereof in a high yield.

### SUMMARY OF THE INVENTION

According to the present invention, a proline compound represented by formula (1) (proline compound (1)), 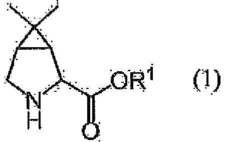 wherein R¹ represents an optionally substituted aliphatic hydrocarbon group or a hydrogen atom,
or a salt thereof is produced by the following steps A to D:
[Step A]
   a step of reacting a pyrrolidinone compound represented by formula (2) (pyrrolidinone compound (2)), 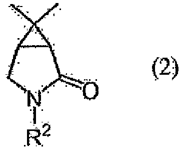 wherein R² represents a group represented by C(R¹⁰)₂Ar, Ar is an optionally substituted phenyl group or an optionally substituted naphthyl group, and two R^{10'}s are each independently a hydrogen atom, an optionally substituted alkyl group or an optionally substituted phenyl group,
   with a reducing agent represented by formula (3) (reducing agent (3)),

   (M^{s+}) _{1/s} H₄₋ₘAl⁻(OR³)ₘ

   wherein R³ represents an optionally substituted alkyl group, M^{s+} represents a metal ion, s represents a valence of the metal ion, and m represents 1, 2 or 3,
   in the presence of a base to obtain a pyrrolidinol compound represented by the following formula (4) (pyrrolidinol compound (4)) or a salt thereof;
[Step B]
   a step of reacting the pyrrolidinol compound represented by formula (4), 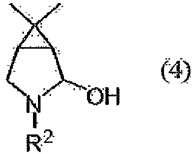 wherein R² represents the same meaning as described above, or the salt thereof with a cyanating agent to obtain a cyanopyrrolidine compound represented by the following formula (5) (cyanopyrrolidine compound (5)) or a salt thereof;
[Step C]
   a step of hydrolyzing or alcoholyzing the cyanopyrrolidine compound represented by formula (5), 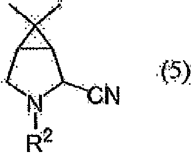
wherein R² represents the same meaning as described above, or the salt thereof to obtain a protected proline compound represented by the following formula (6) (protected proline compound (6)) or a salt thereof; and [Step D]:
   a step of substituting R² in the protected proline compound represented by formula (6), 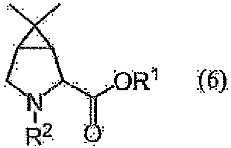 wherein R¹ and R² each represent the same meanings as described above,
   or the salt thereof by a hydrogen atom.

### MODE FOR CARRYING OUT THE INVENTION

Hereinafter, the present invention will be described in more detail.

The "aliphatic hydrocarbon group" in R¹ is usually an aliphatic hydrocarbon group having 1 to 10 carbon atoms, and examples thereof include alkyl groups having 1 to 10 carbon atoms, alkenyl groups having 2 to 10 carbon atoms and alkynyl groups having 2 to 10 carbon atoms, cycloalkyl groups having 4 to 10 carbon atoms, and preferably a methyl group and an ethyl group.

Here, the alkyl group having 1 to 10 carbon atoms includes straight-chain or branched alkyl groups having 1 to 10 carbon atoms such as a methyl group, an ethyl group, a propyl group, an isopropyl group, a butyl group, a sec-butyl group, a tert-butyl group, a pentyl group, a hexyl group, a heptyl group, an octyl group, a nonyl group and a decyl group, and preferably a methyl group and an ethyl group.

The alkenyl group having 2 to 10 carbon atoms includes straight-chain or branched alkenyl groups having 2 to 10 carbon atoms such as a vinyl group, an allyl group, a butenyl group, a pentenyl group, a hexenyl group, a heptenyl group, an octenyl group, a nonenyl group and a decenyl group, and preferably an allyl group and a 2-butenyl group.

The alkynyl group having 2 to 10 carbon atoms includes straight-chain or branched alkynyl groups having 2 to 10 carbon atoms such as an ethynyl group, a propargyl group, a butynyl group, a pentynyl group, a hexynyl group, a heptynyl group, an octynyl group, a nonyl group and a decynyl group, and preferably a propargyl group and a 2-butynyl group.

The cycloalkyl group having 4 to 10 carbon atoms includes a cyclobutyl group, a cyclopentyl group, a cyclohexyl group, a cycloheptyl group, a cyclooctyl group, a cyclononyl group, a cyclodecyl group, and the like.

The aliphatic hydrocarbon group represented by R¹ may have one or more substituents, and examples of the substituent include a hydroxyl group, a halogen atom (fluorine atom, chlorine atom, bromine atom, iodine atom), a nitro group, an alkoxy group and a cycloalkoxy group.

Examples of the alkoxy group include straight-chain or branched alkoxy groups having 1 to 4 carbon atoms such as a methoxy group, an ethoxy group, a propoxy group, an isopropoxy group and a butoxy group.

Examples of the cycloalkoxy group include straight-chain or branched cycloalkoxy groups having 4 to 10 carbon atoms such as a cyclobutyloxy group, a cyclopentyloxy group, a cyclohexyloxy group, a cycloheptyloxy group, a cyclooctyloxy group, a cyclononyloxy group and a cyclodecyloxy group.

R² is a group represented by C(R¹⁰)₂Ar, and this C (carbon atom) binds to a nitrogen atom of a pyrrolidinone ring.

Examples of the substituent of the optionally substituted phenyl group represented by Ar include alkyl groups having 1 to 10 carbon atoms, a hydroxy group, a halogen atom (fluorine atom, chlorine atom, bromine atom, iodine atom), a nitro group, a halogen-substituted alkyl group having 1 to 10 carbon atoms, an alkoxy group, a cycloalkoxy group, a phenyl group, a nitrophenyl group and alkoxyphenyl groups. Here, examples of the alkyl group having 1 to 10 carbon atoms are the same as those described above, and examples of the halogen-substituted alkyl group are those in which one or more hydrogen atoms of the alkyl group are substituted by a halogen atom or atoms. Examples of the alkoxy group are the same as those of the alkoxy group having 1 to 4 carbon atoms described above, and examples of the cycloalkoxy group are the same as those of the cycloalkoxy group having 4 to 10 carbon atoms described above. In addition, the alkoxyphenyl group is a phenyl group having one or more alkoxy group having 1 to 4 carbon atoms as described above, and examples include a methoxyphenyl group, a dimethoxyphenyl group and an ethoxyphenyl group. The preferred optionally substituted phenyl group is a phenyl group, an alkoxyphenyl group and a methylphenyl group.

Examples of the substituent of the optionally substituted naphthyl group represented by Ar are the same as the examples of the substituent of the optionally substituted phenyl group.

Two R¹⁰'s each independently represent a hydrogen atom, an optionally substituted alkyl group (for example, an optionally substituted alkyl group having 1 to 6 carbon atoms), or an optionally substituted phenyl group, and preferably a hydrogen atom, an alkyl group having 1 to 6 carbon atoms or a phenyl group. Here, examples of the substituent in the
optionally substituted alkyl group include a hydroxyl group, a halogen atom (fluorine atom, chlorine atom, bromine atom, iodine atom), a nitro group, an alkoxy group and a cycloalkoxy group, as the same as the examples of the substituent of the aliphatic hydrocarbon group represented by R¹. In addition, examples of the substituent in the optionally substituted phenyl group include the same as the substituent of the optionally substituted phenyl group represented by Ar.

Ar and R¹⁰ in the group represented by C(R¹⁰)₂Ar of R² are described above, and R¹ is preferably a benzyl group, a methoxybenzyl group and an α-phenetyl group, and particularly preferably a benzyl group.

According to the production method of the present invention, by undergoing four steps including steps A to D, the proline compound represented by the formula (1) or the salt thereof can be produced in an excellent yield. Hereinafter, the four steps will be described in detail.

### [Step A]

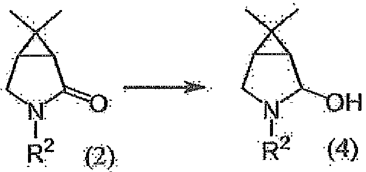

wherein R² represents the same meaning as described above.

Step A is a step of reacting a pyrrolidinone compound (2) with a reducing agent (3) to produce a pyrrolidinol compound (4) or a salt thereof.

Examples of the metal ion represented by M^{s+} in the reducing agent (3) used in the step A include an ion of an alkali metal (lithium, sodium, potassium, and the like) and an ion of alkaline earth metal (magnesium, calcium, and the like), and preferably include lithium ion, sodium ion and potassium ion. In addition, examples of the optionally substituted alkyl group represented by R³ in the reducing agent (3) are the same as the examples of the optionally substituted alkyl group having 1 to 10 carbon atoms as described in the aliphatic hydrocarbon group represented by R¹.

The pyrrolidinone compound (2) can be easily prepared from a commercially available compound. For example, the pyrrolidinone compound (2) can be prepared by N-protecting a lactam compound obtained by the method described in US2009/118519, according to an ordinary method. Alternatively, it can also be prepared according to step E described below. In addition, the pyrrolidinone compound (2) has an asymmetric carbon, and can be present as an optically active substance or a racemic form based on the asymmetric carbon. When the optically active pyrrolidinone compound (2) is used, the optical purity is maintained throughout the steps A to D.

Examples of the pyrrolidinone compound (2) include compounds represented by the following formulae (2-1) to (2-28) and optically active substances of the compounds represented by formulae (2-1) to (2-28). Here, in the specific examples of the pyrrolidinone compound (2) shown here, "Me" refers to a methyl group.

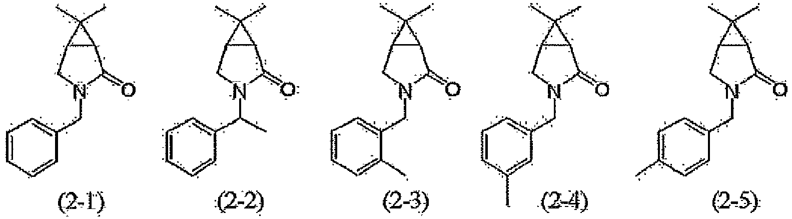

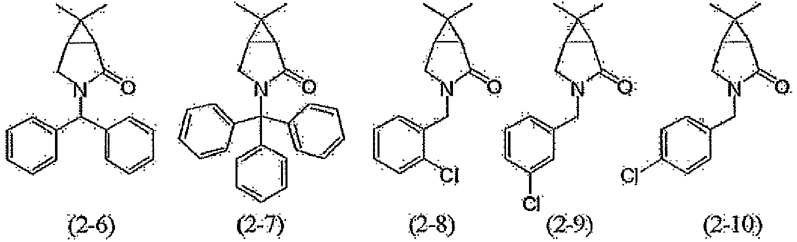

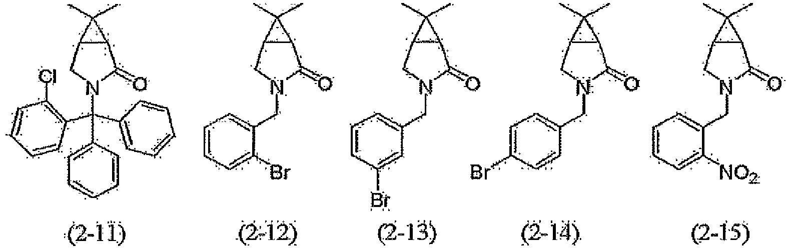

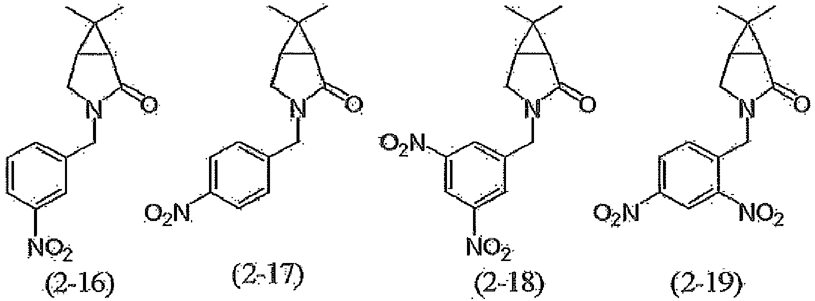

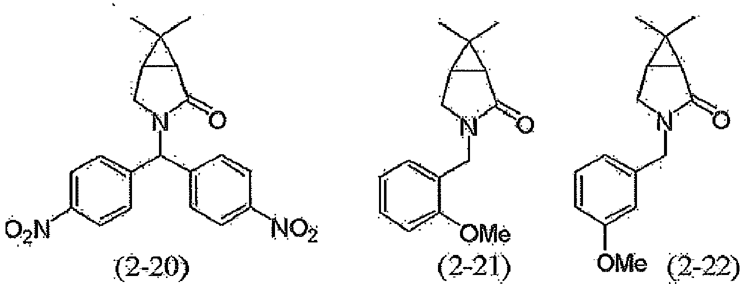

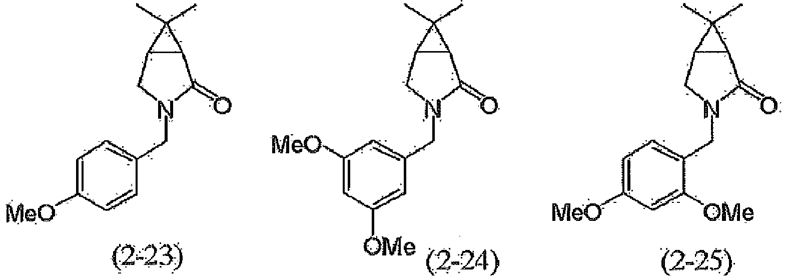

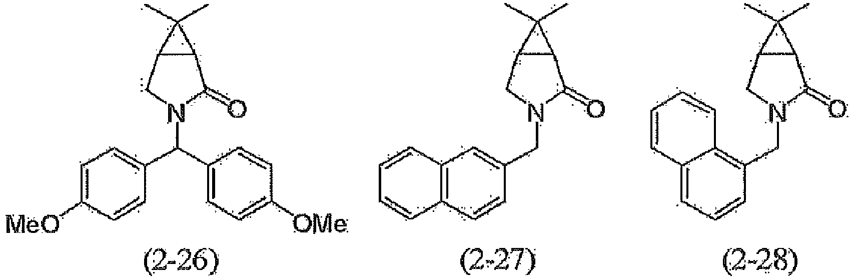

Among the pyrrolidinone compounds (2) represented by formulae (2-1) to (2-28), the pyrrolidinone compounds (2) each represented by formula (2-1), formula (2-2), formula (2-21), formula (2-22) and formula (2-23) are preferable, and the pyrrolidinone compound (2) represented by formula (2-1) is further more preferable.

The reducing agent (3) used in the step A is preferably a reducing agent represented by a formula,

**(M^{s+})**_{1/s}**H₄₋ₘAl⁻[O(CH₂)ₙOR⁴]ₘ**

wherein R⁴ represents an alkyl group or a cycloalkyl group, and n, M^{s+}, s and m each represent the same meanings as described above,
and more preferably it is sodium bis (2-methoxyethoxy) aluminum hydride.

When the pyrrolidinone compound (2) is reduced using a reducing agent such as diisobutylaluminum hydride, the reduction does not stop at the stage of the pyrrolidinol compound (4), and an over-reduced form is much obtained as a by-product. However, when the reducing agent (3) is used, by-production of the over-reduced form is decreased. 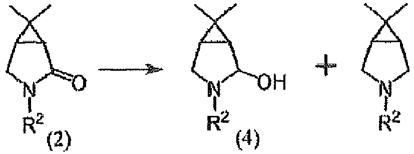 (Over-reduced form)
wherein R² represents the same meaning as described above.

Specific examples of the alkyl group and cycloalkyl group represented by R⁴ are the same as the alkyl groups having 1 to 10 carbon atoms and cycloalkyl groups having 4 to 10 carbon atoms described above regarding the aliphatic hydrocarbon group represented by R¹.

The amount of the reducing agent (3) used is, in the case of the reducing agent (3) having one hydride in a molecule [the reducing agent (3), wherein m is 3], for example, 0.8 to 10 mol, preferably 1 to 6 mol, more preferably 1.1 to 4 mol and further more preferably 1.2 to 3 mol, based on 1 mol of the pyrrolidinone compound (2). In the case of the reducing agent (3) having two hydrides in a molecule [the reducing agent (3), wherein m is 2], the amount of the reducing agent (3) used is, for example, 0.4 to 5 mol, preferably 0.55 to 2.5 mol, more preferably 0.5 to 2 mol and further more preferably 0.6 to 1.5 mol, based on 1 mol of the pyrrolidinone compound (2). In the case of the reducing agent (3) having three hydrides in a molecule [the reducing agent (3), wherein m is 1], the amount of the reducing agent (3) used is, for example, 0.27 to 3.3 mol, preferably 0.33 to 2 mol, more preferably 0.37 to 1.3 mol and further more preferably 0.4 to 1 mol, based on 1 mol of the pyrrolidinone compound (2). Here, it is effective to add a base for suppressing by-production of the over-reduced form.

Examples of the base used in the step A include an alkyl metal alkoxide (potassium tert-butoxide, sodium ethoxide, sodium methoxide, lithium ethoxide, lithium methoxide, and the like), an alkaline earth metal alkoxide (calcium methoxide, magnesium methoxide, and the like), an alkali metal hydroxide (lithium hydroxide, sodium hydroxide, potassium hydroxide, and the like), an alkaline earth metal hydroxide (calcium hydroxide, magnesium hydroxide, and the like), an alkali metal carbonate (lithium carbonate, sodium carbonate, potassium carbonate, and the like), an alkaline earth metal carbonate (calcium carbonate, magnesium carbonate, and the like), and an amine having two or more nitrogen atoms or having one or more oxygen atoms [N,N'-dimethylethylenediamine, N,N,N',N",N"-pentamethyldiethylenetriamine, bis(2-dimethylaminoethyl)ether, bis(2-methoxyethyl)amine, 1-(2-dimethylaminoethyl)-4-methylpiperazine, bis(2-morpholinoethyl)ether], preferably include an alkyl metal alkoxide and an amine having two or more nitrogen atoms, and further preferably include potassium tert-butoxide, sodium ethoxide, sodium methoxide and N,N'-dimethylethylenediamine. The amount of the base used is, for example, 0.01 to 10 mol, preferably 0.1 to 3 mol, and more preferably 0.1 to 0.5 mol, based on 1 mol of the pyrrolidinone compound (2).

The step A is preferably carried out in a solvent.

Examples of the solvent include an aliphatic hydrocarbon (hexane, heptane, and the like), an aromatic hydrocarbon (benzene, toluene, xylene, ethylbenzene, chlorobenzene, dichlorobenzene, and the like), a halogenated hydrocarbon (dichloromethane, dichloroethane, chloroform, and the like), an ether (tetrahydrofuran, dioxane, dimethoxyethane, diethylene glycol dimethyl ether, methyl tert-butyl ether, diethyl ether, and the like) and a mixture thereof. In addition, a small amount of an alcohol (methanol, ethanol, 2-propanol, and the like) can be added to the aliphatic hydrocarbon, aromatic hydrocarbon and the like described above. Preferred solvents include an aliphatic hydrocarbon and an ether, and more preferably include toluene, diethylene glycol dimethyl ether, tetrahydrofuran, dioxane and dimethoxyethane. When ether, particularly, a highly coordinating solvent such as an ether having two or more oxygen atoms, is used, the generation of the over-reduced form is suppressed. The amount of the solvent used is, for example, 1 to 50-fold amount, preferably 1.5 to 20-fold amount, and more preferably 2 to 10-fold amount, based on the weight of the pyrrolidinone compound (2).

The reaction temperature is, for example, -80 to 60°C, preferably -40°C to 40°C, more preferably -30°C to 30°C, and further more preferably -20°C to 20°C. When the reaction temperature is lower, by-production of the over-reduced form is suppressed. The reaction time can be appropriately selected by confirming the progress of reaction and the amount of by-product, and is, for example, 30 minutes to 40 hours, and preferably 1 hour to 10 hours.

After the completion of the reaction of the step A, it is preferred that the reaction mixture containing the pyrrolidinol compound (4) be subjected to a subsequent step B as it is, but the reaction mixture can also be subjected to a post-treatment to isolate the pyrrolidinol compound (4). For example, the reaction mixture and water are mixed to hydrolyze the reducing agent, and as necessary, separated by adding an organic solvent separable from water, whereby an inorganic component in which the reducing agent is hydrolyzed can be distributed to the aqueous layer, and the pyrrolidinol compound (4) can be distributed to the organic layer. When the inorganic component is precipitated, the inorganic component may be removed by filtration operation.

The water to be mixed with the reaction mixture may be neutral water, a basic aqueous solution, or an acidic aqueous solution. Preferably, an aqueous solution containing an inorganic base (basic aqueous solution) such as an alkali metal hydroxide (sodium hydroxide, potassium hydroxide, or the like), an alkali metal carbonate (sodium carbonate, potassium carbonate, or the like), an alkali metal bicarbonate (sodium hydrogen carbonate, potassium hydrogen carbonate, or the like), an alkaline earth hydroxide (magnesium hydroxide, calcium hydroxide, or the like) or an alkaline earth metal carbonate (magnesium carbonate, calcium carbonate, or the like) is used.

In addition, when the reaction mixture is mixed with water or a basic aqueous solution, it may be further mixed with a chelating agent. Examples of the chelating agent include sodium gluconate, sodium tartrate, potassium sodium tartrate, ethylenediaminetetraacetic acid, diethylenetriaminepentaacetic acid, and nitrilotriacetic acid. The chelating agent preferably includes sodium gluconate. Separation efficiency can be improved by mixing with such a chelating agent.

Examples of the acid when using the acidic aqueous solution include hydrogen chloride, hydrogen bromide, sulfuric acid and phosphoric acid.

The amount of each of the water, base, acid and chelating agent used is not particularly limited. The amount used can be an amount required for dissolving the inorganic substance generated by hydrolysis of the reducing agent, or only an amount required for hydrolysis of the reducing agent may be used and the generated inorganic component may be removed by filtration operation.

Examples of the organic solvent separable from water described above include an aliphatic hydrocarbon (hexane, heptane, cyclohexane, and the like), an aromatic hydrocarbon (toluene, xylene, monochlorobenzene, dichlorobenzene, and the like), an ether (methyl tert-butyl ether and the like), a halogenated hydrocarbon (dichloromethane, dichloroethane, chlorobutane, and the like), a ketone (methyl isobutyl ketone and the like) and an ester (ethyl acetate, butyl acetate, and the like).

The organic layer obtained by separation may be further subjected to washing with water, washing with a basic aqueous solution, or washing with an acidic aqueous solution. In addition, an appropriate inorganic salt may be dissolved in water to be used for washing with water. Washing with water, washing with a basic aqueous solution, washing with an acidic aqueous solution or the like may be repeatedly carried out.

The organic layer containing the obtained pyrrolidinol compound (4) is subjected to condensation, distillation, recrystallization, column chromatography purification or the like, whereby the pyrrolidinol compound (4) can also be isolated and purified. In the recrystallization, an inorganic acid such as hydrogen chloride, hydrogen bromide, phosphoric acid, sulfuric acid, nitric acid or boric acid, or an organic acid such as acetic acid, citric acid, oxalic acid, methanesulfonic acid, toluenesulfonic acid or trifluoromethane sulfonic acid is used, whereby the pyrrolidinol compound (4) can also be collected as an acid addition salt.

Examples of the pyrrolidinol compound (4) include compounds represented by the following formulae (4-1) to (4-28), optically active substances of the compounds represented by formulae (4-1) to (4-28), and acid addition salts thereof.

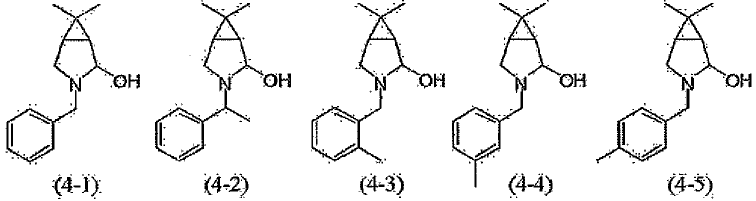

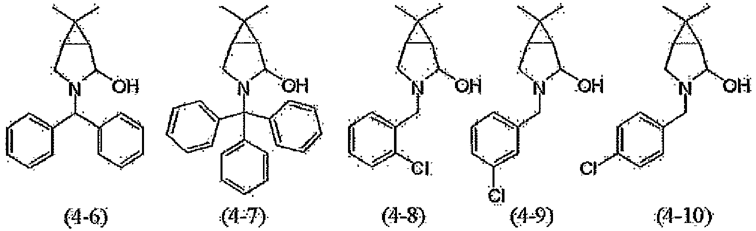

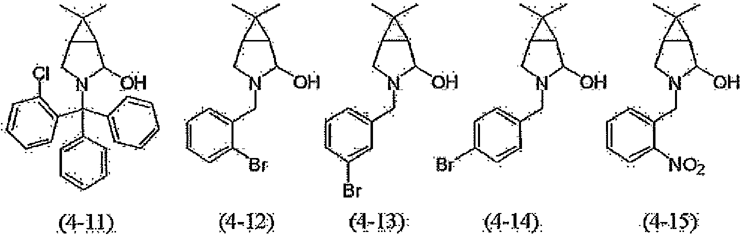

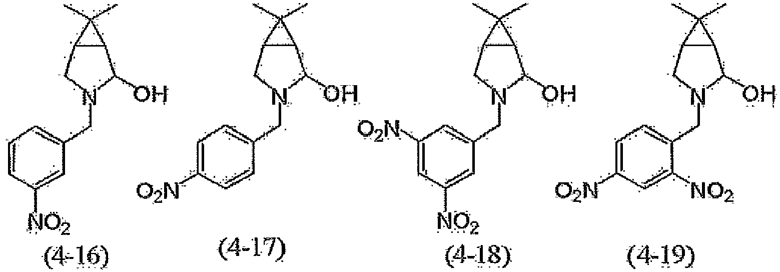

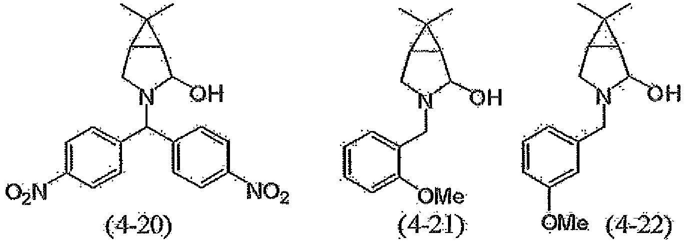

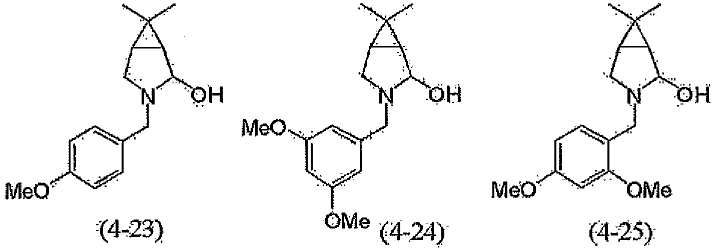

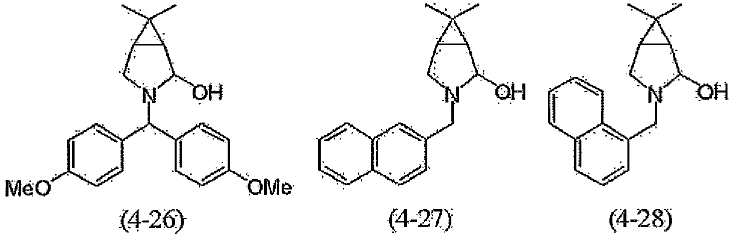

### [Step B]

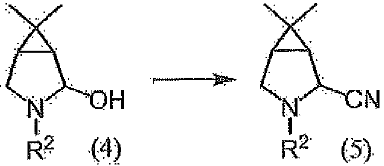

wherein R² represents the same meaning as described above.

Step B is a step of reacting the pyrrolidinol compound (4) obtained in the step A or the salt thereof with a cyanating agent. A cyanopyrrolidine compound (5) or a salt thereof is produced by carrying out the step B.

In the case where the reaction mixture obtained in the step A is subjected to the step B as it is, while the reaction mixture obtained in the step A is mixed with the cyanating agent, it may be further mixed with water, an acid or an alcohol. Water, an acid or an alcohol can be each mixed and used, and further can be mixed with the above-described chelating agent and used.

The water to be mixed with the reaction mixture may be neutral water, a basic aqueous solution, or an acidic aqueous solution.

As the basic aqueous solution, for example, an aqueous solution containing an inorganic base such as an alkali metal hydroxide (sodium hydroxide, potassium hydroxide, or the like), an alkali metal carbonate (sodium carbonate, potassium carbonate, or the like), an alkali metal bicarbonate (sodium hydrogen carbonate, potassium hydrogen carbonate, or the like), an alkaline earth metal hydroxide (magnesium hydroxide, calcium hydroxide, or the like) or an alkaline earth metal carbonate (magnesium carbonate, calcium carbonate, or the like) is used.

When the acidic aqueous solution is used, examples of the acid include an inorganic acid (hydrochloric acid, hydrobromic acid, sulfuric acid, phosphoric acid, and the like) and an organic acid (acetic acid, citric acid, p-toluenesulfonic acid, and the like), and preferably include hydrochloric acid and acetic acid.

Examples of the alcohol for mixing include methanol, ethanol, 1-propanol, 2-propanol, 1-butanol and 2-methyl-2-propanol, and preferably include methanol.

The amount of each of the water, alcohol, acid and chelating agent used is not particularly limited.

Examples of the cyanating agent include a metal cyanide (alkali metal cyanides such as sodium cyanide and potassium cyanide, alkaline earth metal cyanides such as calcium cyanide and magnesium cyanide, and the like) and hydrogen cyanide, and preferably include sodium cyanide and potassium cyanide. These cyanating agents may be used in the state of a solution dissolved in water or an organic solvent. The amount of the cyanating agent used includes 0.8 to 30 mol, preferably 1 to 5 mol, and more preferably 1.2 to 3 mol, based on 1 mol of the theoretical amount of the pyrrolidinol compound (4) in the reaction mixture obtained in the step A. The amount of the pyrrolidinol compound (4) in the reaction mixture obtained in the step A can be determined by an appropriate analysis means to set the amount of the cyanating agent used.

It is also preferred to add an acid. Examples of the acid to be added include an inorganic acid (hydrochloric acid, hydrobromic acid, sulfuric acid, phosphoric acid, and the like) and an organic acid (acetic acid, citric acid, p-toluenesulfonic acid, and the like), preferably include an organic acid, and more preferably include acetic acid. The amount of the acid used is 0.01 to 30 mol, preferably 0.1 to 10 mol, and more preferably 0.2 to 5 mol, based on 1 mol of the theoretical amount of the pyrrolidinol compound (4).

The solvent used in the step A can be used as it is, and the amount used in the step A is also preferably used.

The reaction temperature is, for example, -80°C to 100°C, preferably -20°C to 30°C, and more preferably 0°C to 5°C. The reaction time can be appropriately selected by confirming the progress of reaction and the amount of by-product, and is, for example, 30 minutes to 40 hours, and preferably 1 hour to 20 hours.

After the completion of the reaction of the pyrrolidinol compound (4) with the cyanating agent, a reaction mixture containing a cyanopyrrolidine compound (5) is obtained. This reaction mixture can be subj ected to a post-treatment according to an ordinary method.

For example, as necessary, water, or water and an organic solvent separable from water are added, and the mixture is separated, whereby an excess cyanating agent can be distributed to the aqueous layer. In addition, the cyanopyrrolidine compound (5) can be distributed to the organic layer. Furthermore, when the pH during post-treatment is alkaline, the cyanating agent, the reducing agent, and the inorganic components formed by hydrolysis thereof can be efficiently distributed to the aqueous layer, and it is thus preferred that, after mixing the reaction mixture and water during the post-treatment, the mixture be made alkaline by adding a base thereto, or a basic aqueous solution be used as water to be mixed with the reaction mixture. The range of the pH in the post-treatment operation is, for example, the range of 8 to 14, and preferably the range of 11 to 13.

Examples of the base used in the post-treatment include an inorganic base, for example, an alkali metal hydroxide such as sodium hydroxide and potassium hydroxide, an alkali metal carbonate such as sodium carbonate and potassium carbonate, and an alkali metal bicarbonate such as sodium hydrogen carbonate and potassium hydrogen carbonate.

Examples of the organic solvent separable from water described above include an aliphatic hydrocarbon (hexane, heptane, cyclohexane, and the like), an aromatic hydrocarbon (toluene, xylene, monochlorobenzene, dichlorobenzene, and the like), an ether (methyl tert-butyl ether and the like), a halogenated hydrocarbon (dichloromethane, dichloroethane, chlorobutane, and the like), a ketone (methyl isobutyl ketone and the like) and an ester (ethyl acetate, butyl acetate, and the like).

The obtained organic layer may be further subjected to washing with water, washing with a basic aqueous solution or an acidic aqueous solution, or the like. In addition, an appropriate inorganic salt may be dissolved in water to be used for washing with water. Washing with water, washing with a basic aqueous solution or an acidic aqueous solution or the like may be repeatedly carried out.

A post-treatment may be carried out using sodium hypochlorite or hydrogen peroxide to decompose the cyanating agent.

The organic layer containing the obtained cyanopyrrolidine compound (5) is also subjected to condensation, distillation, recrystallization, column chromatography purification or the like, whereby the cyanopyrrolidine compound (5) can also be isolated and purified. In the recrystallization, an inorganic acid such as hydrogen chloride, hydrogen bromide, phosphoric acid, sulfuric acid, nitric acid or boric acid, or an organic acid such as acetic acid, citric acid, oxalic acid, methanesulfonic acid, toluenesulfonic acid or trifluoromethane sulfonic acid is used, whereby the cyanopyrrolidine compound (5) can also be collected as an acid addition salt.

Examples of the cyanopyrrolidine compound (5) include compounds represented by the following formulae (5-1) to (5-28), optically active substances of the compounds represented by formulae (5-1) to (5-28) and acid addition salts thereof. Here, when an optically active (1R,5S) isomer is used as the pyrrolidinone compound (2) in the present steps A and B, an optically active (1R,2S,5S) isomer is obtained as the cyanopyrrolidine compound (5). On the other hand, when an optically active (1S,5R) isomer is used as the pyrrolidinone compound (2) in the present steps A and B, an optically active (1S,2R,5R) isomer is obtained as the cyanopyrrolidine compound (5). The stereoisomerism is also maintained in the following steps C and D. A compound derived from the optically active

(1R,2S,5S) isomer of the cyanopyrrolidine compound (5) herein referred, specifically, the compound represented by the following formula (1b), is a particularly useful compound as an intermediate compound of a medicinal or agrochemical agent. 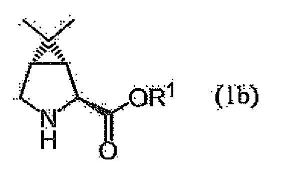 wherein R¹ represents the same meaning as described above.

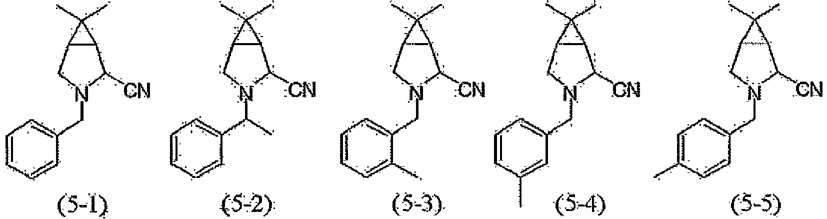

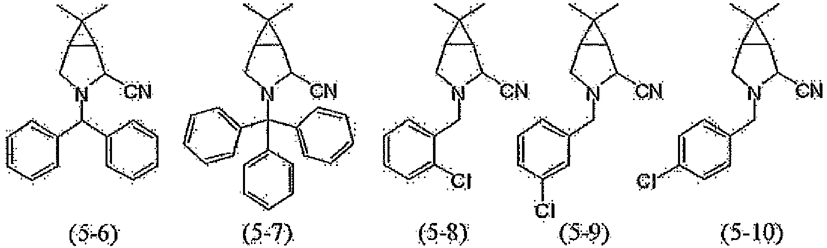

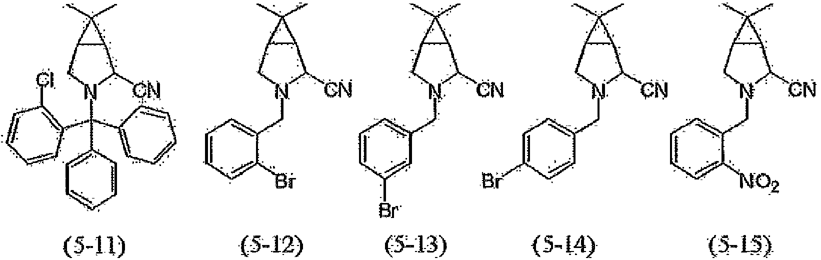

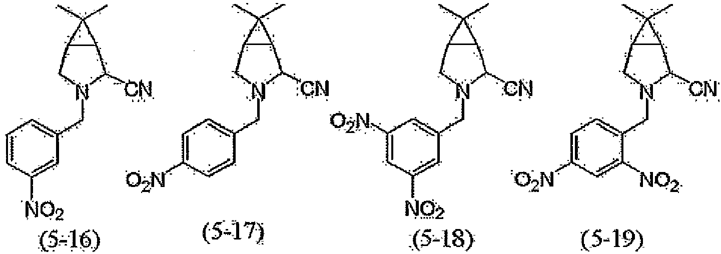

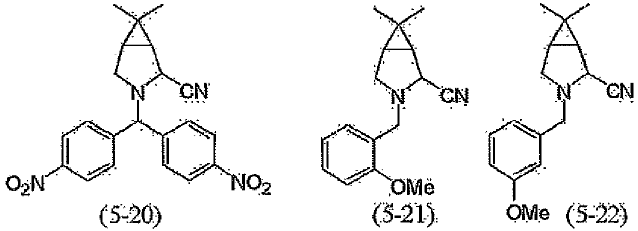

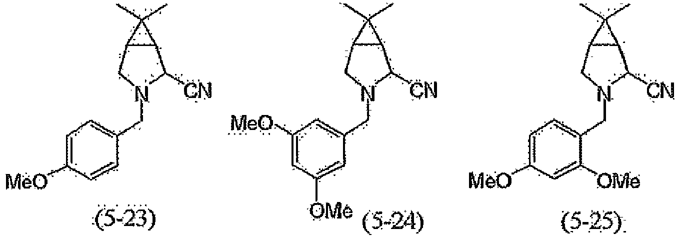

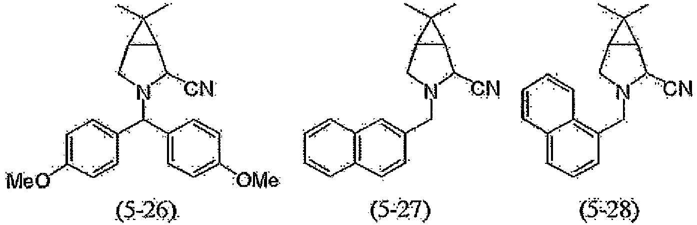

### [Step C]

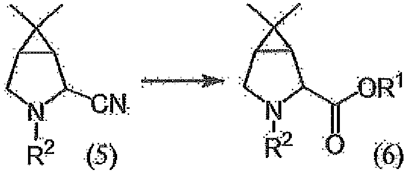

wherein R¹ and R² represent the same meanings as described above.

Step C is a step of hydrolyzing or alcoholyzing the cyanopyrrolidine compound (5) obtained in the step B or the salt thereof. A protected proline compound (6) or a salt thereof is produced by carrying out the step C. For the hydrolysis or alcoholysis in the step C, a compound represented by a formula,

**R¹-OH**

wherein R¹ represents the same meaning as described above, is used.

The used amount of the compound represented by formula R¹-OH is, for example, 1 mol or more based on 1 mol of the cyanopyrrolidine compound (5). The upper limit thereof is not limited, and the compound can also be excessively used as a solvent. The used amount of the compound represented by formula R¹-OH is preferably 1.5 to 50 mol based on 1 mol of the cyanopyrrolidine compound (5).

The reaction of the cyanopyrrolidine compound (5) with the compound represented by formula R¹-OH in the step C is, for example, carried out in the presence of an acid.

Examples of the acid thereof include hydrogen chloride, hydrogen bromide, sulfuric acid, acetic acid, methanesulfonic acid and toluenesulfonic acid, and preferably include hydrogen chloride. The amount of the acid used is, for example, an amount so that the concentration in the reaction mixture is 1 to 60% by weight, and preferably an amount so that the concentration in the reaction mixture is 20 to 55% by weight. During the reaction, the acid may be appropriately added to maintain the concentration described above.

The reaction in the step C is also preferably carried out in a solvent. Examples of the solvent include an aliphatic hydrocarbon (hexane, heptane, and the like), an aromatic hydrocarbon (benzene, toluene, xylene, ethylbenzene, chlorobenzene, dichlorobenzene, and the like), a halogenated hydrocarbon (dichloromethane, dichloroethane, chloroform, and the like), an ether (tetrahydrofuran, dioxane, dimethoxyethane, diethylene glycol dimethyl ether, methyl tert-butyl ether, diethyl ether, and the like), an alcohol (methanol, ethanol, 2-propanol, and the like), an ester (ethyl acetate, propyl acetate, and the like) and a mixture thereof. The amount of the solvent used is, for example, 1 to 100-fold amount, preferably 1.1 to 20-fold amount, and more preferably 1.5 to 10-folk amount, based on the weight of the cyanopyrrolidine compound (5).

The reaction temperature is, for example, -80°C to 100°C, and preferably -40°C to 65°C. The reaction time can be appropriately selected by confirming the progress of reaction and the amount of by-product, and is, for example, 30 minutes to 40 hours, and preferably 1 hour to 20 hours.

The protected proline compound (6) can be collected by, as necessary, adding an organic solvent separable from water to the reaction mixture obtained by the reaction of the cyanopyrrolidine compound (5) with the compound represented by formula R¹-OH to distribute the protected proline compound (6) to the organic layer.

Examples of the organic solvent separable from water include an aliphatic hydrocarbon (hexane, heptane, cyclohexane, and the like), an aromatic hydrocarbon (toluene, xylene, monochlorobenzene, dichlorobenzene, and the like), an ether (methyl tert-butyl ether and the like), a halogenated hydrocarbon (dichloromethane, dichloroethane, chlorobutane, and the like), a ketone (methyl isobutyl ketone and the like) and an ester (ethyl acetate, butyl acetate, and the like).

The obtained organic layer may be further subjected to washing with water, washing with a basic aqueous solution or an acidic aqueous solution, or the like. In addition, an appropriate inorganic salt may be dissolved in water to be used for washing with water. Washing with water, washing with a basic aqueous solution or an acidic aqueous solution or the like may be repeatedly carried out.

The organic layer containing the obtained protected proline compound (6) is subj ected to condensation, distillation, recrystallization, column chromatography purification or the like, whereby the protected proline compound (6) can also be isolated and purified. In the recrystallization, an inorganic acid such as hydrogen chloride, hydrogen bromide, phosphoric acid, sulfuric acid, nitric acid or boric acid, or an organic acid such as acetic acid, citric acid, oxalic acid, methanesulfonic acid, toluenesulfonic acid or trifluoromethane sulfonic acid is used, whereby the protected proline compound (6) can also be collected as an acid addition salt.

The step C can also be carried out through an imidate compound represented by formula (7) (imidate compound (7)), 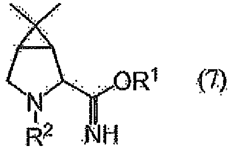 wherein R¹ and R² each represent the same meanings as described above,
or a tautomer thereof.

That is, in the step C, the imidate compound (7) is obtained from the cyanopyrrolidine compound (5), and the protected proline compound (6) can also be produced from the obtained imidate compound (7).

An acid is allowed to act on the cyanoproline compound (5), thereby obtaining, as the imidate compound (7), an amide compound represented by formula (7a) (amide compound (7a)) that is a tautomer thereof, 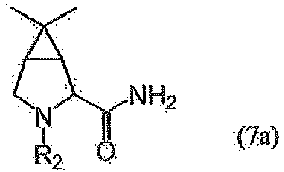 wherein R² represents the same meaning as described above, or a salt thereof, and the obtained amide compound (7a) is reacted with a compound represented by a formula,

**R¹-OH**

wherein R¹ represents the same meaning as described above, thereby producing a protected proline compound (6) or a salt thereof.

Examples of the acid include hydrogen chloride (hydrogen chloride gas, hydrochloric acid), hydrogen bromide (hydrogen bromide gas, hydrobromic acid), sulfuric acid, phosphoric acid, citric acid and acetic acid, and preferably include hydrogen chloride gas and hydrochloric acid. Also, an acid chloride and an alcohol can be combined and used for the acid.

Examples of the acid chloride include acetyl chloride and oxalyl chloride, and preferably include acetyl chloride.

The amount of the acid or acid chloride used is, for example, 1 to 30 mol, and preferably 3 to 20 mol, based on 1 mol of the theoretical amount of the cyanoproline compound (5).

The reaction in the step C that undergoes the imidate compound (7) is also preferably carried out in a solvent. Examples of the solvent include an aliphatic hydrocarbon (hexane, heptane, and the like), an aromatic hydrocarbon (benzene, toluene, xylene, ethylbenzene, chlorobenzene, dichlorobenzene, and the like), a halogenated hydrocarbon (dichloromethane, dichloroethane, chloroform, and the like), an ether (tetrahydrofuran, dioxane, dimethoxyethane, diethylene glycol dimethyl ether, methyl tert-butyl ether, diethyl ether, and the like), an alcohol (methanol, ethanol, 2-propanol, and the like), an ester (ethyl acetate, propyl acetate, and the like), water, and a mixture thereof. The amount of the solvent used is, for example, 1 to 100-fold amount, preferably 1.1 to 20-fold amount, and more preferably 1.5 to 10-fold amount, based on the weight of the cyanopyrrolidine compound (5).

The reaction temperature is, for example, -30°C to 50°C, preferably -10°C to 30°C, and more preferably 0°C to 30°C.

The reaction time can be appropriately selected by confirming the progress of reaction and the amount of by-product, and is, for example, 30 minutes to 40 hours, and preferably 1 hour to 10 hours.

The protected proline compound (6) may be produced in the course of the reaction, but can be converted to the amide compound (7a) by appropriately selecting the reaction time.

After the completion of the reaction, the obtained reaction mixture may be subj ected to a post-treatment to isolate the amide compound (7a), and when water is not used as a solvent, the obtained reaction mixture may be reacted with the compound represented by formula R¹-OH, thereby producing the protected proline compound (6). When the obtained reaction mixture may be subjected to a post-treatment, for example, as necessary, an organic solvent separable from water is mixed with the reaction mixture, and the amide compound (7a) is distributed to the organic layer, whereby the amide compound (7a) can be collected. In addition, as necessary, the mixture during post-treatment is made alkaline, whereby the amide compound (7a) can also be efficiently distributed to the organic layer.

Examples of the organic solvent separable from water include an aliphatic hydrocarbon (hexane, heptane, cyclohexane, and the like), an aromatic hydrocarbon (toluene, xylene, monochlorobenzene, dichlorobenzene, and the like), an ether (methyl tert-butyl ether and the like), a halogenated hydrocarbon (dichloromethane, dichloroethane, chlorobutane, and the like), a ketone (methyl isobutyl ketone and the like) and an ester (ethyl acetate, butyl acetate, and the like).

The organic layer obtained by separation may be further subjected to washing with water, washing with a basic aqueous solution or an acidic aqueous solution, or the like. In addition, an appropriate inorganic salt may be dissolved in water to be used for washing with water. Washing with water, washing with a basic aqueous solution or an acidic aqueous solution or the like may be repeatedly carried out.

The organic layer containing the obtained amide compound (7a) is subjected to condensation, distillation, recrystallization, column chromatography purification or the like, whereby the amide compound (7a) can also be isolated and purified. In the recrystallization, an inorganic acid such as hydrogen chloride, hydrogen bromide, phosphoric acid, sulfuric acid, nitric acid or boric acid, or an organic acid such as acetic acid, citric acid, oxalic acid, methanesulfonic acid, toluenesulfonic acid or trifluoromethane sulfonic acid is used, whereby the amide compound (7a) can also be obtained as an acid addition salt.

Examples of the amide compound (7a) include compounds represented by the following formulae (7a-1) to (7a-28), acid addition salts of compounds the represented by the formulae (7a-1) to (7a-28), and optically active substances thereof.

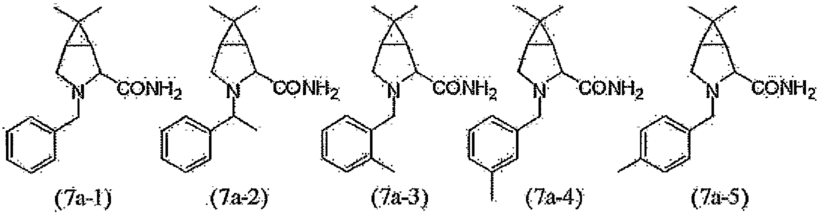

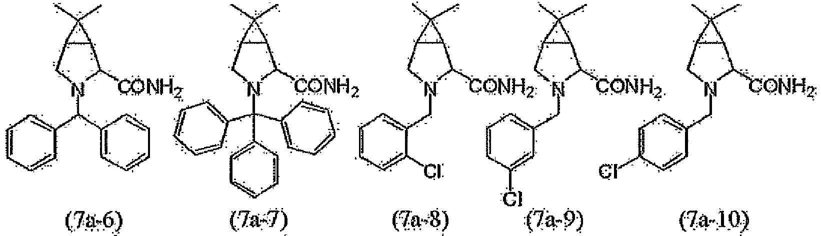

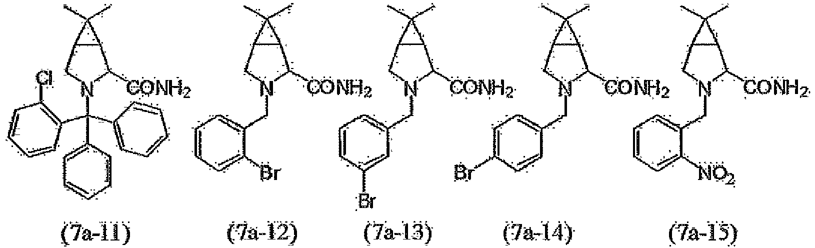

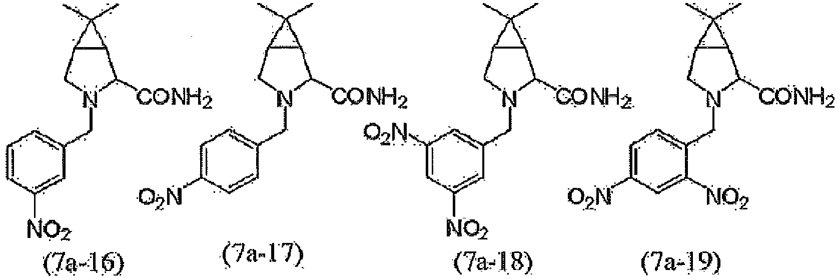

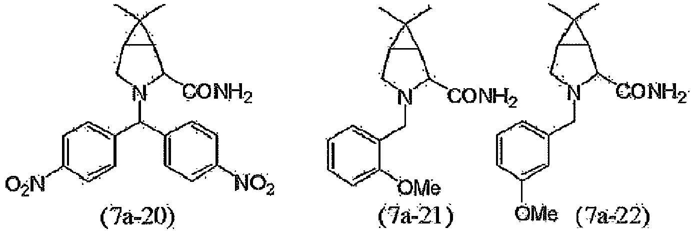

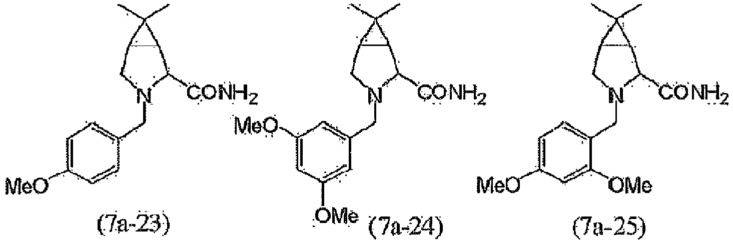

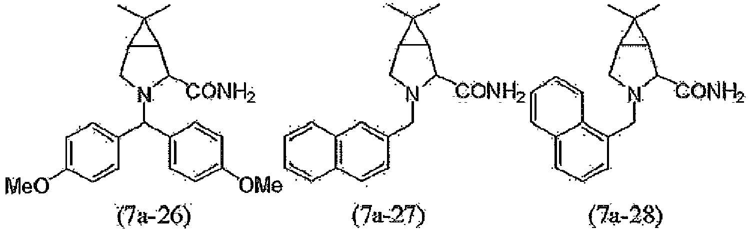

A protected proline compound (6) or a salt thereof is obtained by a reaction of the amide compound (7a) with the compound represented by formula R¹-OH in the presence of at least one selected from the group consisting of acids and acid chlorides.

The used amount of the compound represented by formula R¹-OH is, for example, 1 mol or more based on 1 mol of the amide compound (7a). The upper limit thereof is not limited, and the compound can also be used as a solvent. The used amount of the compound represented by formula R¹-OH is preferably 1 to 50 mol and more preferably 1 to 10 mol, based on 1 mol of the amide compound (7a).

Examples of the acid include hydrogen chloride, hydrogen bromide, sulfuric acid, phosphoric acid and methanesulfonic acid, and preferably include methanesulfonic acid and sulfuric acid.

The amount of the acid used is, for example, 1 to 20 mol and preferably 5 to 10 mol, based on 1 mol of the amide compound (7a).

Examples of the acid chloride include acetyl chloride, thionyl chloride, sulfuryl chloride, phosphorus trichloride, phosphorus pentachloride and oxalyl chloride, and preferably include thionyl chloride.

The amount of the acid chloride used is 1 to 20 mol and preferably 1 to 3 mmol, based on 1 mol of the amide compound (7a).

The reaction temperature is, for example, 0°C to 100°C, preferably 40°C to 80°C, and more preferably 60°C to 70°C. The reaction time can be appropriately selected by confirming the progress of reaction and the amount of by-product, and is, for example, 30 minutes to 40 hours, and preferably 1 hour to 20 hours.

The reaction mixture containing the protected proline compound (6) obtained by the reaction of the amide compound (7a) with the compound represented by formula R¹-OH may be subjected to a post-treatment. For example, as necessary, an organic solvent separable fromwater is mixed with the reaction mixture, and the protected proline compound (6) is distributed to the organic layer, whereby the protected proline compound (6) can be collected. In addition, as necessary, the mixture during post-treatment is made alkaline, whereby the protected proline compound (6) can also be efficiently distributed to the organic layer.

Examples of the organic solvent separable from water include an aliphatic hydrocarbon (hexane, heptane, cyclohexane, and the like), an aromatic hydrocarbon (toluene, xylene, monochlorobenzene, dichlorobenzene, and the like), an ether (methyl tert-butyl ether and the like), a halogenated hydrocarbon (dichloromethane, dichloroethane, chlorobutane, and the like), a ketone (methyl isobutyl ketone and the like) and an ester (ethyl acetate, butyl acetate, and the like).

The obtained organic layer may be further subjected to washing with water, washing with a basic aqueous solution or an acidic aqueous solution, or the like. In addition, an appropriate inorganic salt may be dissolved in water to be used for washing with water. Washing with water, washing with a basic aqueous solution or an acidic aqueous solution or the like may be repeatedly carried out.

The organic layer containing the obtained protected proline compound (6) is subjected to condensation, distillation, recrystallization, column chromatography purification or the like, whereby the protected proline compound (6) can also be isolated and purified. In the recrystallization, an inorganic acid such as hydrogen chloride, hydrogen bromide, phosphoric acid, sulfuric acid, nitric acid or boric acid, or an organic acid such as acetic acid, citric acid, oxalic acid, methanesulfonic acid, toluenesulfonic acid or trifluoromethane sulfonic acid is used, whereby the protected proline compound (6) can also be collected as an acid addition salt.

Also, an imidate compound (7) can be obtained by a reaction of the cyanoproline compound (5) with the compound represented by formula R¹-OH in the presence of a base, and the obtained imidate compound (7) is mixed with acidic water, whereby a protected proline compound (6) can be produced.

Examples of the base include an alkali metal hydroxide (lithium hydroxide, sodium hydroxide, potassium hydroxide, and the like), an alkaline earth metal hydroxide (calcium hydroxide, magnesium hydroxide, and the like), an alkali metal carbonate (lithium carbonate, sodium carbonate, potassium carbonate, and the like) and an alkaline earth metal carbonate (calcium carbonate, magnesium carbonate, and the like). The amount of the base used is, for example, 0.1 to 20 mol and preferably 0.2 to 10 mol, based on 1 mol of the cyanopyrrolidine compound (5).

The amount of the compound represented by formula R¹-OH used is, for example, 1 mol or more based on 1 mol of the cyanopyrrolidine compound (5). The upper limit thereof is not limited, and the compound can also be used as a solvent. The amount of the compound represented by formula R¹-OH used is preferably 2 to 50 mol based on 1 mol of the cyanopyrrolidine compound (5).

As already described, the reaction that undergoes the imidate compound (7) is also preferably carried out in a solvent. Examples of the solvent include an aliphatic hydrocarbon (hexane, heptane, and the like), an aromatic hydrocarbon (benzene, toluene, xylene, ethylbenzene, chlorobenzene, dichlorobenzene, and the like), a halogenated hydrocarbon (dichloromethane, dichloroethane, chloroform, and the like), an ether (tetrahydrofuran, dioxane, dimethoxyethane, diethylene glycol dimethyl ether, methyl tert-butyl ether, diethyl ether, and the like), an alcohol (methanol, ethanol, 2-propanol, and the like), an ester (ethyl acetate, propyl acetate, and the like) and a mixture thereof. The amount of the solvent used is, for example, 1 to 100-fold amount, preferably 1.1 to 20-fold amount, and more preferably 1.5 to 10-fold amount, based on the weight of the cyanopyrrolidine compound (5).

The reaction temperature is, for example, -50°C to 60°C, preferably -30°C to 30°C, and more preferably -20°C to 10°C. The reaction time can be appropriately selected by confirming the progress of reaction and the amount of by-product, and is, for example, 30 minutes to 40 hours, and preferably 1 hour to 20 hours.

Examples of the imidate compound (7) include compounds represented by the following formulae (7-1) to (7-28) (imino methyl esters), imino ethyl esters, imino propyl esters, imino isopropyl esters, imino butyl esters, imino tert-butyl esters, imino isobutyl esters, imino vinyl esters and imino allyl esters thereof, and optically active substances thereof.

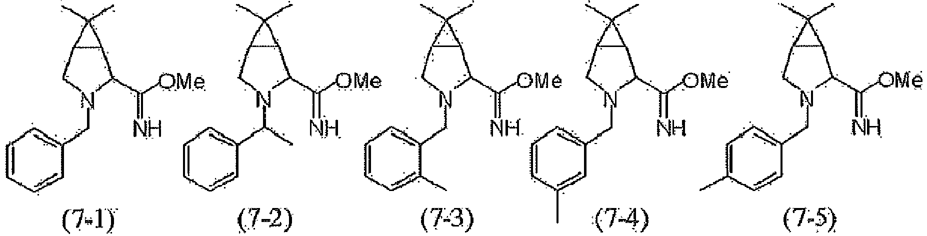

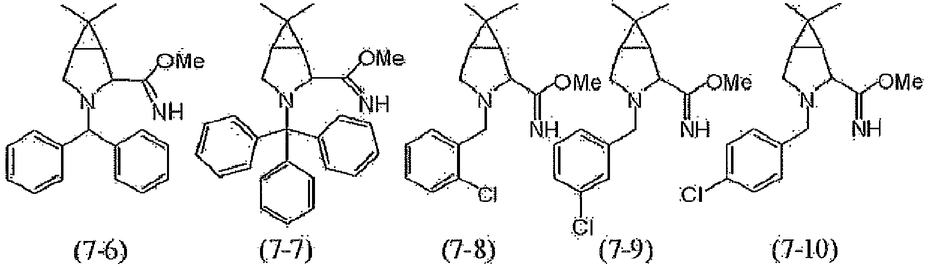

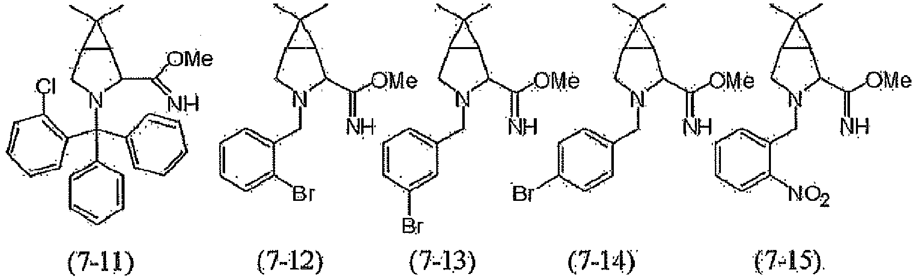

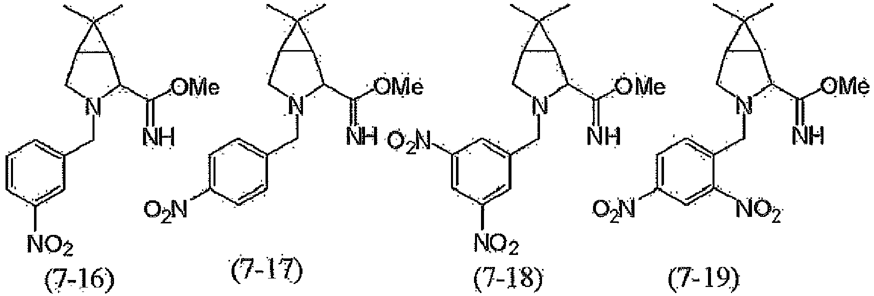

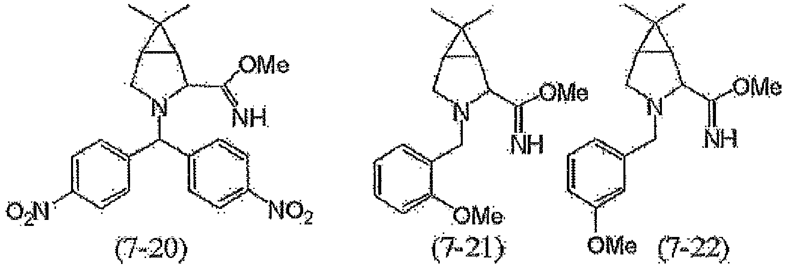

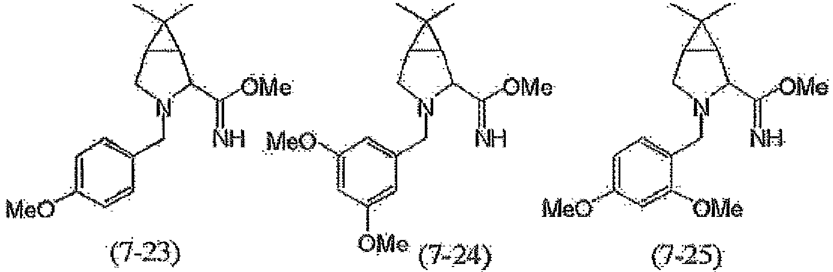

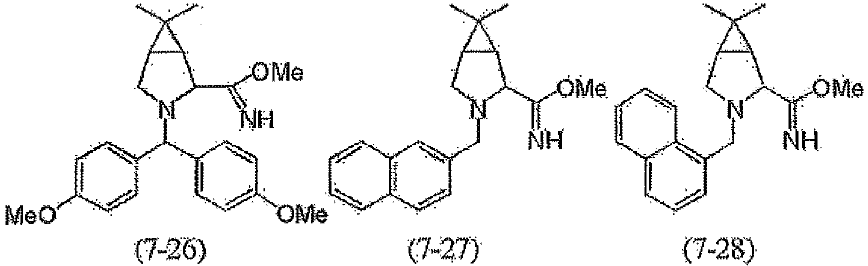

The imidate compound (7) is mixed with an acidic aqueous solution to obtain a protected proline compound (6).

The mixing of the imidate compound (7) with the acidic aqueous solution may be carried out, for example, by adding the acidic aqueous solution to the reaction mixture containing the imidate compound (7), or adding the reaction mixture containing the imidate compound (7) to the acidic aqueous solution. In addition, the mixing may be carried out by mixing the reaction mixture containing the imidate compound (7) with water, and adding the acidic aqueous solution to the obtained mixture.

Examples of the acidic aqueous solution include aqueous solutions of inorganic acids such as hydrochloric acid, hydrobromic acid, sulfuric acid or phosphoric acid and aqueous solutions of organic acids such as acetic acid or citric acid.

The amount of the acid used is, for example, 1 to 20 mol based on 1 mol of the cyanopyrrolidine compound (5). The preferred amount of the acid used is the amount obtained by further adding 0.5 to 2 mol of the acid based on 1 mol of the cyanopyrrolidine compound (5) to the amount of the acid required to neutralize a base used during the reaction to obtain the imidate compound (7).

The imidate compound (7) is mixed with the acidic aqueous solution and then mixed with an organic solvent separable from water as necessary, and the protected proline compound (6) is distributed to the organic layer, whereby the protected proline compound (6) can be collected.

Examples of the organic solvent separable from water include an aliphatic hydrocarbon (hexane, heptane, cyclohexane, and the like), an aromatic hydrocarbon (toluene, xylene, monochlorobenzene, dichlorobenzene, and the like), an ether (methyl tert-butyl ether and the like), a halogenated hydrocarbon (dichloromethane, dichloroethane, chlorobutane, and the like), a ketone (methyl isobutyl ketone and the like) and an ester (ethyl acetate, butyl acetate, and the like).

The obtained organic layer may be further subjected to washing with water, washing with a basic aqueous solution or an acidic aqueous solution, or the like. In addition, an appropriate inorganic salt may be dissolved in water to be used for washing with water. Washing with water, washing with a basic aqueous solution or an acidic aqueous solution or the like may be repeatedly carried out.

The organic layer containing the obtained protected proline compound (6) is further subjected to condensation, distillation, recrystallization, column chromatography purification or the like, whereby the protected proline compound (6) can also be isolated and purified. In the recrystallization, an inorganic acid such as hydrogen chloride, hydrogen bromide, phosphoric acid, sulfuric acid, nitric acid or boric acid, or an organic acid such as acetic acid, citric acid, oxalic acid, methanesulfonic acid, toluenesulfonic acid or trifluoromethane sulfonic acid is used, whereby the protected proline compound (6) can also be collected as an acid addition salt.

Examples of the protected proline compound (6) include compounds represented by the following formulae (6-1) to (6-28) (methyl esters), ethyl esters, propyl esters, isopropyl esters, butyl esters, tert-butyl esters, isobutyl esters, vinyl esters and allyl esters thereof, optically active substances thereof, and acid addition salts thereof.

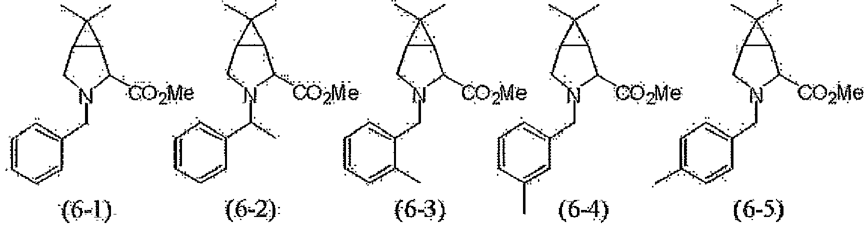

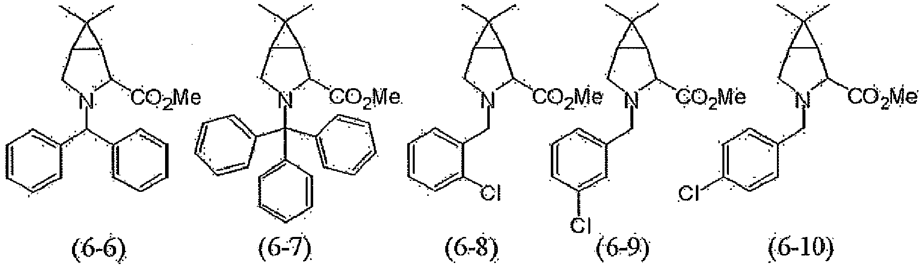

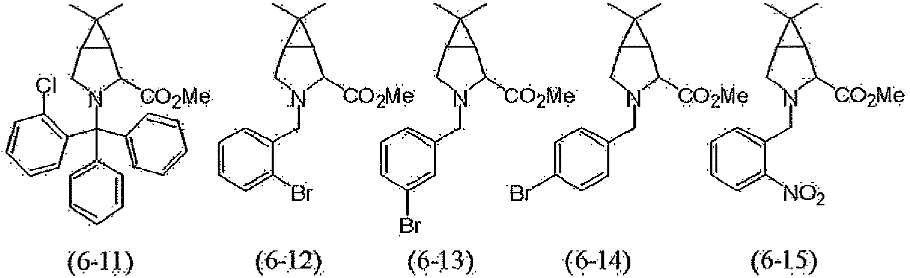

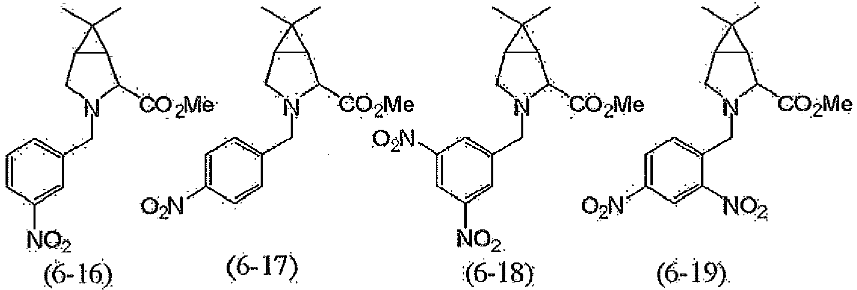

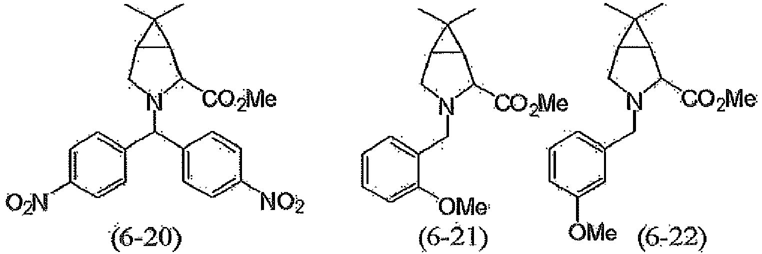

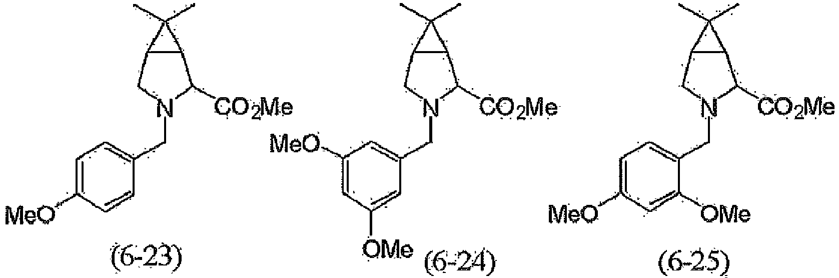

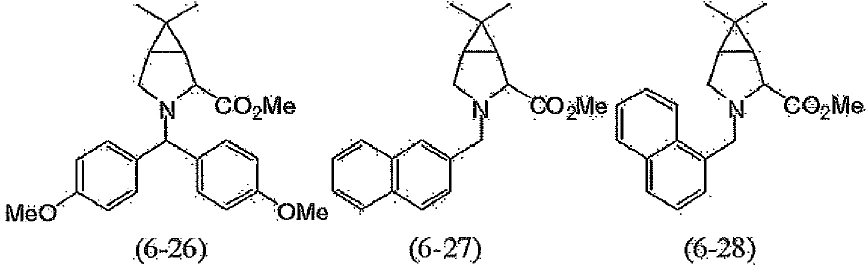

### [Step D]

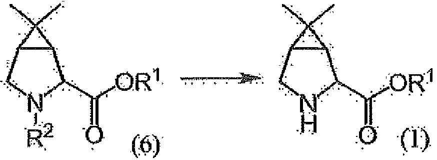

wherein R¹ and R² represent the same meanings as described above.

Step D is a step of substituting R² in the protected proline compound (6) obtained in the step C or the salt thereof by a hydrogen atom. A proline compound (1) or a salt thereof is produced by carrying out the step D.

To substitute R² of the protected proline compound (6) by a hydrogen atom, an ordinary method to deprotect an N-protecting group such as a benzyl group is adopted. Hereinafter, substitution of the group represented by R² by a hydrogen atom may be referred to as deprotection or deprotection reaction. Deprotection can be carried out by reduction treatment of the protected proline compound (6) (hydrogenolysis by hydrogen/reduction catalyst, reduction treatment by a reducing agent such as sodium metal/liquid ammonia, reduction treatment by electrolytic reduction, or the like), and can also be performed by acid-treating the protected proline compound (6).

For example, when carrying out hydrogenolysis, it can be carried out by stirring under an atmosphere of hydrogen gas using palladium, palladium/carbon, platinum, Raney nickel or the like as a reduction catalyst. Examples of the solvent when carrying out hydrogenolysis include an aliphatic hydrocarbon (hexane, heptane, and the like), an aromatic hydrocarbon (benzene, toluene, xylene, ethylbenzene, chlorobenzene, dichlorobenzene, and the like), a halogenated hydrocarbon (dichloromethane, dichloroethane, chloroform, and the like), an ether (tetrahydrofuran, dioxane, dimethoxyethane, diethylene glycol dimethyl ether, methyl tert-butyl ether, diethyl ether, and the like), an alcohol (methanol, ethanol, 2-propanol, and the like), an ester (ethyl acetate, propyl acetate, and the like), a nitrile (acetonitrile and the like), water, and a mixture thereof. The amount of the solvent used is, for example, 1 to 50-fold amount, preferably 1.5 to 20-fold amount, and more preferably 2 to 10-fold amount, based on the weight of the protected proline compound (6).

The reaction temperature in the deprotection reaction is within the range of, usually -20°C to 100°C, and preferably 0°C to 50°C. The reaction time can be appropriately selected by confirming the progress of reaction and the amount of by-product.

After the deprotection reaction, an insoluble solid such as reduction catalyst can be removed by filtration. After the deprotection reaction, the reaction mixture containing the obtained proline compound (1) may be subjected to a post-treatment. For example, the proline compound (1) can be collected by mixing the reaction mixture obtained after the deprotection reaction, as necessary, water or a basic aqueous solution, and an organic solvent separable from water, and extracting the proline compound (1) into the organic layer.

Examples of the organic solvent separable from water that is used during separation include an aliphatic hydrocarbon (hexane, heptane, cyclohexane, and the like), an aromatic hydrocarbon (toluene, xylene, monochlorobenzene, dichlorobenzene, and the like), an ether (methyl tert-butyl ether and the like), a halogenated hydrocarbon (dichloromethane, dichloroethane, chlorobutane, and the like), a ketone (methyl isobutyl ketone and the like) and an ester (ethyl acetate, butyl acetate, and the like).

The organic layer obtained by separation may be further subjected to washing with water, washing with a basic aqueous solution or an acidic aqueous solution, or the like. In addition, an appropriate inorganic salt may be dissolved in water to be used for washing with water. Washing with water, washing with a basic aqueous solution or an acidic aqueous solution or the like may be repeatedly carried out.

The organic layer containing the obtained proline compound (1) is subjected to condensation, distillation, recrystallization, column chromatography purification or the like, whereby the proline compound (1) can also be isolated and purified. In the recrystallization, an inorganic acid such as hydrogen chloride, hydrogen bromide, phosphoric acid, sulfuric acid, nitric acid or boric acid, or an organic acid such as acetic acid, citric acid, oxalic acid, methanesulfonic acid, toluenesulfonic acid or trifluoromethane sulfonic acid is used, whereby the proline compound (1) can also be collected as an acid addition salt.

In addition, the reaction mixture obtained after the deprotection reaction is mixed with an acid or poor solvent as necessary, whereby the proline compound (1) can also be obtained as a crystal. Examples of the acid include an inorganic acid such as hydrogen chloride gas, hydrogen bromide, phosphoric acid, hydrochloric acid, sulfuric acid, nitric acid and boric acid, and an organic acid such as acetic acid, citric acid, oxalic acid, methanesulfonic acid, toluenesulfonic acid and trifluoromethane sulfonic acid, and preferably include hydrogen chloride gas and hydrochloric acid. Examples of the poor solvent include an organic solvent such as an aliphatic hydrocarbon (hexane, heptane, cyclohexane, and the like), an aromatic hydrocarbon (toluene, xylene, monochlorobenzene, dichlorobenzene, and the like), an ether (tetrahydrofuran, methyl tert-butyl ether, 1,4-dioxane, 1,2-dimethoxyethane, and the like), a halogenated hydrocarbon (dichloromethane, dichloroethane, chlorobutane, and the like) and an ester (methyl acetate, ethyl acetate, butyl acetate, and the like). These organic solvents may be one kind or a mixture of two or more kinds.

The amount of the organic solvent used is within the range of, for example, 0 to 200-fold, and preferably 1 to 50-fold, based on the weight of the proline compound (1).

The obtained proline compound (1) may also be further purified by the method such as recrystallization.

Examples of the proline compound (1) include, for example, compounds represented by the following formulae (1-1) to (1-10), optically active substances of the compounds represented by formulae (1-1) to (1-10), and acid addition salts thereof.

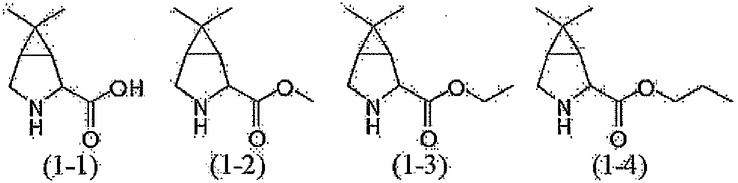

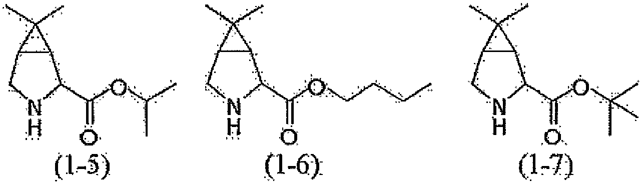

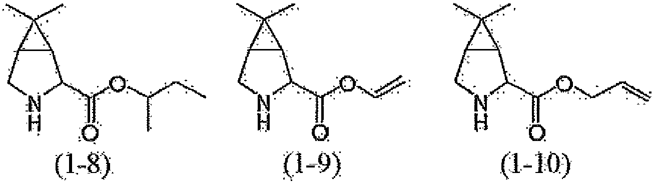

The pyrrolidinone compound (2) used in the step A can also be produced by the following step E.

### [Step E]

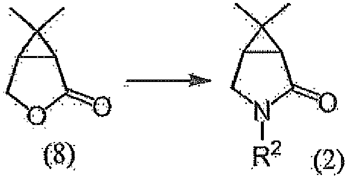

wherein R² represents the same meaning as described above.

Step E is a step of reacting a compound represented by formula (8) with a compound represented by formula (9),

**H₂N-R²**

wherein R² represents the same meaning as described above. A pyrrolidinone compound (2) is produced by carrying out the step E.

The lactone compound (8) can be easily prepared from a commercially available compound. For example, the lactone compound (8) can be prepared according to the method described in US2010/168463.

In the step E, the amount of the amine compound (9) used is within the range of, for example, 0.8 to 10 mol, preferably 1 to 6 mol, more preferably 1.1 to 4 mol and further more preferably 1.7 to 3 mol, based on 1 mol of the lactone compound (8).

The reaction of the lactone compound (8) with the amine compound (9) is carried out in the absence of a solvent, or in the presence of water or an organic solvent. Examples of the solvent include an aliphatic hydrocarbon (hexane, heptane, cyclohexane, and the like), an aromatic hydrocarbon (benzene, toluene, xylene, ethylbenzene, chlorobenzene, dichlorobenzene, and the like), a halogenated hydrocarbon (dichloromethane, dichloroethane, chloroform, chlorobutane, and the like), an ether (tetrahydrofuran, dioxane, dimethoxyethane, diethylene glycol dimethyl ether, methyl tert-butyl ether, diethyl ether, and the like), an alcohol (methanol, ethanol, 1-propanol, 2-propanol, 1-butanol, 2-methyl-2-propanol, and the like), and a nitrile (acetonitrile, propionitrile, and the like). The organic solvent may be a mixture of two or more kinds, and may be a mixture with water. The solvent that can be used in the reaction of the lactone compound (8) with the amine compound (9) has been described above, and among them, it is preferred that the reaction of the lactone compound (8) with the amine compound (9) be carried out in a mixed solvent of water and an organic solvent.

The amount of water used is within the range of, for example, 0.1 to 100-fold, and preferably 0.2 to 30-fold, based on the weight of the lactone compound (8).

The amount of the organic solvent used is within the range of, for example, 0 to 100-fold, and preferably 0.1 to 20-fold, based on the weight of the lactone compound (8).

The reaction of the lactone compound (8) with the amine compound (9) may be carried out, for example, in the presence of an additive selected from an inorganic acid (hydrogen chloride, hydrogen bromide, hydrofluoric acid, sulfuric acid, nitric acid, phosphoric acid, boric acid, and the like), an organic acid (formic acid, acetic acid, citric acid, oxalic acid, methanesulfonic acid, toluenesulfonic acid, trifluoromethane sulfonic acid, and the like), an acidic ion-exchange resin, γ-Al₂O₃, N-hydroxybenzotriazole and tetrabutylammonium bromide. Preferred additives are toluenesulfonic acid and γ-Al₂O₃.

The reaction temperature of the lactone compound (8) with the amine compound (9) is, for example, within the range of 100°C to 300°C, preferably 100°C to 200°C, more preferably 150°C to 200°C, and further more preferably 160°C to 185°C.

The reaction of the lactone compound (8) with the amine compound (9) may be carried out in a pressurized condition. The pressure carried out in the pressurized condition is within the range of, for example, 0.1 MPa to 5 MPa, and preferably D.5 MPa to 2 MPa.

After the completion of the reaction of the lactone compound (8) with the amine compound (9), a reaction mixture containing the pyrrolidinone compound (2) is obtained. The obtained reaction mixture is mixed with water, then further mixed with an organic solvent separable from water as necessary, and separated, thereby distributing an unreacted amine compound (9), an additive, and components generated by decomposing these substances to the aqueous layer, and thus the pyrrolidinone compound (2) can be collected as the organic layer. The obtained organic layer may be further subjected to washing with water, washing with basic water or washing with acidic water, and washing with water, washing with basic water, washing with acidic water or the like may be repeatedly carried out.

The organic layer containing the pyrrolidinone compound (2) may be used in the next step as it is, or the pyrrolidinone compound (2) is isolated by distilling off the solvent or the like, and the isolated pyrrolidinone compound (2) may be used in the next step. The pyrrolidinone compound (2) may be purified by a method such as column chromatography purification or recrystallization, and then used in the next step.

### EXAMPLES

Hereinafter, the present invention will be described in further detail with reference to Examples.

### Example 1

### [Step A]: Synthesis of (1R,5S)-3-benzyl-6,6-dimethyl-3-azabicyclo[3.1.0]hexan-2-ol (Compound A)

(1R,5S)-3-Benzyl-6,6-dimethyl-3-azabicyclo[3.1.0]hexa n-2-one (64.6 g) (300 mmol) (Compound E), 6.70 g (59.7 mmol) of potassium tert-butoxide and 129 g of toluene were mixed and then cooled to -11°C. Thereto was added dropwise 69.3 g of a toluene solution (70% weight concentration, 240 mmol) of sodium bis(2-methoxyethoxy)aluminum hydride at -10°C over 1 hour and 20 minutes, and the mixture was stirred at the same temperature for 2 hours and 20 minutes. The reaction mixture was added dropwise to a mixed solution of 195 g of a 2% aqueous sodium hydroxide solution and 9.8 g of sodium gluconate at 10°C over 20 minutes. Thereafter, the mixture was allowed to stand still and separated, and the resulting organic layer was washed with 130 g of a 2% aqueous sodium hydroxide solution twice, with 66 g of a 2% aqueous sodium hydroxide solution once, and with 66 g of 5% saline once. The resulting organic layer was dried over 11 g of anhydrous magnesium sulfate, and concentrated under reduced pressure conditions to distill off the solvent, thereby obtaining 62.4 g of white crystals containing 61.2 g (282 mmol: yield of 93.9%)^{*)} of Compound A.
¹H-NMR(CDC₃)δ: 7.3-7.26(5H, m), 4.18(1H, s), 3.87(1H, d), 3.75(1H, d), 3.10(1H, dd), 2.62(1H, d), 1.35-1.28(2H, m), 1.08(3H, s), 0.96(3H, s)
*) quantitatively analyzed using HPLC in the following conditions.

### [HPLC Conditions]

Column: CAPCELLPAC C18 MGIII, 4.6 mmφ × 100 mm, 3 µm (manufactured by Shiseido Co., Ltd.)

Flow rate: 1.35 ml/min

Detection wavelength: UV 220 nm

Mobile phase: Solution A (0.1% phosphoric acid)/Solution B (acetonitrile)

### Gradient conditions:

| | | |
|---|---|---|
| Time (min.) | 0 | 45 |
| Solution A (%) | 95 | 30 |
| Solution B (%) | 5 | 70 |

Column temperature: 35°C

### Example 2

### [Step A]: Synthesis of Compound A

Compound E (10.0 g) (46.4 mmol), 0.50 g (9.25 mmol) of sodium methoxide and 20 g of toluene were mixed and then cooled to 10°C. Thereto was added dropwise 8.72 g of a toluene solution (70% weight concentration, 30.2 mmol) of sodium bis(2-methoxyethoxy)aluminum hydride at 10°C over 2 hours, and the mixture was stirred at the same temperature for 3 hours. This reaction mixture liquid was analyzed by HPLC. Here, HPLC conditions are the same as the HPLC conditions in Example 1.

Conversion rate from raw materials to product: 99.8%

Conversion rate (%): (Compound A + Over-reduced form)/(Compound A + Over-reduced form + Raw materials) × 100

The inversion rate (%) is calculated in the same manner also in other Examples.

Ratio of Compound A to (1R,5S)-3-benzyl-6,6-dimethyl-3-azabicyclo[3.1.0]hexane (over-reduced form): 100.0/0.0

HPLC area percentage of Compound A: 98.9%

### Example 3

### [Step A]: Synthesis of Compound A

Compound E (1.00 g) (4.64 mmol), 0.10 g (0.89 mmol) of potassium tert-butoxide and 2. 0 g of tetrahydrofuran were mixed and then cooled to -10°C. Thereto was added dropwise 1.37 g of a toluene solution (70% weight concentration, 4.74 mmol) of sodium bis(2-methoxyethoxy)aluminum hydride at -10°C over 20 minutes, and the mixture was stirred at the same temperature for 25 hours. The reaction mixture liquid was analyzed by gas chromatography. GC conditions for analysis were as follows.

Conversion rate from raw materials to product: 98.3%

Ratio of Compound A to over-reduced form: 99.4/0.6

GC area percentage of Compound A: 96.6%

### [GC Conditions]

Column :DB-1701, 0.25 mmφ × 30 m, 0.25 µm (J&W Scientific, Inc.)

Detection method: FID

Flow rate: 1 ml/min (helium)

### Column temperature:

| | | | | |
|---|---|---|---|---|
| Time (min.) | 0 | 10 | 46 | 55 |
| Temperature (°C) | 100 | 100 | 280 | 280 |

Injector temperature: 150°C

Detector temperature: 280°C

### Example 4

### [Step A]: Synthesis of Compound A

Compound E (1.00 g) (4.64 mmol), 100 mg (0.89 mmol) of potassium tert-butoxide and 2.0 g of methyl tert-butyl ether were mixed and then cooled to -10°C. Thereto was added dropwise 1.09 g of a toluene solution (70% weight concentration, 3.77 mmol) of sodium bis(2-methoxyethoxy) aluminum hydride at -10°C over 20 minutes, and the mixture was stirred at the same temperature for 22 hours. This reaction mixture solution was analyzed by gas chromatography. GC conditions for analysis were the same as the GC conditions in Example 3.

Conversion rate from raw materials to product: 99.2%

Ratio of Compound A to over-reduced form: 99.8/0.2

GC area percentage of Compound A: 97.7%

### Example 5

### [Step A]: Synthesis of Compound A

Compound E (1.00 g) (4.64 mmol), 0.10 g (0.89 mmol) of potassium tert-butoxide and 2.0 g of tetrahydrofuran were mixed and then cooled to -10°C. Thereto was added dropwise 1.09 g of a toluene solution (70% weight concentration, 3.77 mmol) of sodium bis(2-methoxyethoxy)aluminum hydride at -10°C over 20 minutes, and the mixture was stirred at the same temperature for 1 hour. This reaction mixture liquid was analyzed by gas chromatography. GC conditions for analysis were the same as the GC conditions in Example 3.

Conversion rate from raw materials to Compound A: 98.7%

Ratio of Compound A to over-reduced form: 99.6/0.4

GC area percentage of Compound A: 97.1%

### [Step A]: Synthesis of Compound A

Compound E (1.50 g) (6.97 mmol), 0.16 g (1.40 mmol) of potassium tert-butoxide and 3.0 g of tetrahydrofuran were mixed and then heated to 20°C. Thereto was added dropwise 1.64 g of a toluene solution (70% weight concentration, 5.68 mmol) of sodium bis(2-methoxyethoxy)aluminum hydride at 20°C over 20 minutes, and the mixture was stirred at the same temperature for 25 hours. This reaction mixture liquid was analyzed by gas chromatography. GC conditions for analysis were the same as the GC conditions in Example 3.

Conversion rate from raw materials to product: 98.1%

Ratio of Compound A to over-reduced form: 99.7/0.3

GC area percentage of Compound A: 96.1%

### Example 7

### [Step A]: Synthesis of Compound A

Compound E (1.50 g) (6.97 mmol), 0.16 g (1.40 mmol) of potassium tert-butoxide and 3.0 g of tetrahydrofuran were mixed and then heated to 40°C. Thereto was added dropwise 1.64 g of a toluene solution (70% weight concentration, 5.68 mmol) of sodium bis(2-methoxyethoxy)aluminum hydride at 40°C over 20 minutes, and the mixture was stirred at the same temperature for 25 hours. This reaction mixture liquid was analyzed by gas chromatography. GC conditions for analysis were the same as the GC conditions in Example 3.

Conversion rate from raw materials to product: 98.2%

Ratio of Compound A to over-reduced form: 99.0/1.0

GC area percentage of Compound A: 88.8%

### Example 8

### [Step A]: Synthesis of Compound A

Compound E (1.50 g) (6.97 mmol), 38 mg (0.70 mmol) of sodium methoxide and 3.0 g of tetrahydrofuran were mixed and then cooled to -10°C. Thereto was added dropwise 1.64 g of a toluene solution (70% weight concentration, 5.68 mmol) of sodium bis(2-methoxyethoxy)aluminum hydride at -10°C over 20 minutes, and the mixture was stirred at the same temperature for 25 hours. This reaction mixture liquid was analyzed by HPLC. HPLC conditions for analysis were the same as the HPLC conditions in Example 1.

Conversion rate from raw materials to product: 99.9%

Ratio of Compound A to over-reduced form: 97.8/2.2

HPLC area percentage of Compound A: 96.7%

### Example 9

### [Step A]: Synthesis of Compound A

Compound E (1.50 g) (6.97 mmol), 75 mg (1.39 mmol) of sodium methoxide and 3.0 g of tetrahydrofuran were mixed and then cooled to -10°C. Thereto was added dropwise 1.64 g of a toluene solution (70% weight concentration, 5.68 mmol) of sodium bis(2-methoxyethoxy)aluminum hydride at -10°C over 20 minutes, and the mixture was stirred at the same temperature for 1 hour. This reaction mixture liquid was analyzed by gas chromatography. GC conditions for analysis were the same as the GC conditions in Example 3.

Conversion rate from raw materials to Compound A: 98.4%

Ratio of Compound A to over-reduced form: 99.2/0.8

GC area percentage of Compound A: 92.7%

### Example 10

### [Step A]: Synthesis of Compound A

Compound E (1.50 g) (6.97 mmol), 151 mg (2.79 mmol) of sodium methoxide and 3.0 g of tetrahydrofuran were mixed and then cooled to -10°C. Thereto was added dropwise 1.64 g of a toluene solution (70% weight concentration, 5.67 mmol) of sodium bis(2-methoxyethoxy)aluminum hydride at -10°C over 20 minutes, and the mixture was stirred at the same temperature for 25 hours. This reaction mixture liquid was analyzed by HPLC. HPLC conditions for analysis were the same as the HPLC conditions in Example 1.

Conversion rate from raw materials to product: 100.0%

Ratio of Compound A to over-reduced form: 99.7/0.3

HPLC area percentage of Compound A: 98.5%

### Example 11

### [Step A]: Synthesis of Compound A

Compound E (1.50 g) (6.97 mmol), 95 mg (1.40 mmol) of sodium methoxide and 3.0 g of tetrahydrofuran were mixed and then cooled to -10°C. Thereto was added dropwise 1.6A g of a toluene solution (70% weight concentration, 5.68 mmol) of sodium bis(2-methoxyethoxy)aluminum hydride at -10°C over 20 minutes, and the mixture was stirred at the same temperature for 25 hours. This reaction mixture liquid was analyzed by HPLC. HPLC conditions for analysis were the same as the HPLC conditions in Example 1.

Conversion rate from raw materials to product: 99.8%

Ratio of Compound A to over-reduced form: 99.7/0.3

HPLC area percentage of Compound A: 98.1%

### Example 12

### [Step A]: Synthesis of Compound A

Compound E (0.43 g (content of 99.3%, 1.86 mmol)), 0.17 g (1.93 mmol) of N,N'-dimethylethylenediamine and 3.7 g of tetrahydrofuran were mixed and then cooled to -10°C. Thereto was added dropwise 0.86 g of a toluene solution (70% weight concentration, 2.98 mmol) of sodium bis (2-methoxyethoxy) aluminum hydride at -10°C over 20 minutes, and the mixture was stirred at the same temperature for 22 hours. Thereafter, thereto was added dropwise 0.59 g of a toluene solution (70% weight concentration, 2.04 mmol) of sodium bis (2-methoxyethoxy) aluminum hydride at -10°C, and the mixture was stirred at the same temperature for 3 hours. This reaction mixture liquid was analyzed by gas chromatography. GC conditions for analysis were the same as the GC conditions in Example 3.

Conversion rate from raw materials to Compound A: 95.3%

Ratio of Compound A to over-reduced form: 100/0

GC area percentage of Compound A: 95.3%

### Example 13

### [Step A]: Synthesis of Compound A

Compound E (1.00 g) (4.64 mmol), 0.40 g (2.32 mmol) of N,N,N',N",N"-pentamethyldiethylenetriamine and 2.0 g of tetrahydrofuran were mixed and then cooled to -10°C. Thereafter, thereto was added dropwise 2.19 g of a toluene solution (70% weight concentration, 7.58 mmol) of sodium bis (2-methoxyethoxy) aluminum hydride at -10°C over 20 minutes, and the mixture was stirred at the same temperature for 25 hours. This reaction mixture liquid was analyzed by gas chromatography. GC conditions for analysis were the same as the GC conditions in Example 3.

Conversion rate from raw materials to product: 98.3%

Ratio of Compound A to over-reduced form: 80/20

GC area percentage of Compound A: 78.3%

### Example 14

### [Step A]: Synthesis of Compound A

Compound E (1.00 g) (4.64 mmol), 0.40 g (2.34 mmol) of 1-(2-dimethylaminoethyl)-4-methylpiperazine and 2.0 g of tetrahydrofuran were mixed and then cooled to -10°C. Thereto was added dropwise 2.19 g of a toluene solution (70% weight concentration, 7.58 mmol) of sodium bis (2-methoxyethoxy)aluminum hydride at -10°C over 20 minutes, and the mixture was stirred at the same temperature for 25 hours. Thereafter, this reaction mixture liquid was analyzed by gas chromatography. GC conditions for analysis were the same as the GC conditions in Example 3.

Conversion rate from raw materials to product: 98.1%

Ratio of Compound A to over-reduced form: 82/18

GC area percentage of Compound A: 79.6%

### Example 15

### [Step A]: Synthesis of Compound A

Compound E (1.00 g) (4.64 mmol), 0.37 g (2.31 mmol) of bis(2- dimethylaminoethyl)ether and 2.0 g of tetrahydrofuran were mixed and then cooled to -10°C. Thereto was added dropwise 2.19 g of a toluene solution (70% weight concentration, 7.58 mmol) of sodium bis (2-methoxyethoxy) aluminum hydride at -10°C over 20 minutes, and after the dropwise addition, the mixture was stirred at the same temperature for 25 hours. This reaction mixture liquid was analyzed by gas chromatography. GC conditions for analysis were the same as the GC conditions in Example 3.

Conversion rate from raw materials to product: 97.9%

Ratio of Compound A to over-reduced form: 85/15

GC area percentage of Compound A: 83.2%

### Example 16

### [Step A]: Synthesis of Compound A

Compound E (1.00 g) (4.64 mmol), 0.56 g (2.29 mmol) of bis (2-morpholinoethyl) ether and 2. 0 g of tetrahydrofuran were mixed and then cooled to -10°C. Thereto was added dropwise 2.19 g of a toluene solution (70% weight concentration, 7.58 mmol) of sodium bis(2-methoxyethoxy)aluminum hydride at -10°C over 20 minutes, and the mixture was stirred at the same temperature for 25 hours. This reaction mixture liquid was analyzed by gas chromatography. GC conditions for analysis were the same as the GC conditions in Example 3.

Conversion rate from raw materials to product: 97.8%

Ratio of Compound A to over-reduced form: 81/19

GC area percentage of Compound A: 77.1%

### Example 17

### [Step A]: Synthesis of Compound A

Compound E (1.00 g) (4.64 mmol), 0.31 g (2.32 mmol) of bis(2-methoxyethyl)amine and 2.0 g of tetrahydrofuran were mixed and then cooled to -10°C. Thereto was added dropwise 2.19 g of a toluene solution (70% weight concentration, 7.58 mmol) of sodium bis(2-methoxyethoxy)aluminum hydride at -10°C over 20 minutes, and the mixture was stirred at the same temperature for 25 hours. This reaction mixture liquid was analyzed by gas chromatography. GC conditions for analysis were the same as the GC conditions in Example 3.

Conversion rate from raw materials to product: 97.9%

Ratio of Compound A to over-reduced form: 94/6

GC area percentage of Compound A: 89.0%

### Example 18

### [Steps A+B] : Synthesis of (1R, 2S, 5S)-3-benzyl-6, 6-dimethyl-3-azabicyclo[3.1.0]hexane-2-carbonitrile (Compound B)

Compound E (20.0 g) (92.9 mmol), 1.00 g (18.5 mmol) of sodium methoxide and 40.0 g of tetrahydrofuran were mixed and then cooled to -10°C. Thereto was added dropwise 21.46 g of a toluene solution (70% weight concentration, 74.3 mmol) of sodium bis(2-methoxyethoxy)aluminum hydride at -10°C over 2 hours, and the mixture was stirred at the same temperature for 21 hours. The reaction mixture was added dropwise to a mixed liquid of 6.35 g (29.1 mmol) of sodium gluconate, 5.46 g (111.4 mmol) of sodium cyanide and 40 g of water at 5°C over 30 minutes, further 1.67 g (27.8 mmol) of acetic acid was added dropwise at 5°C, and the mixture was stirred at the same temperature for 12 hours. Thereafter, 10 g of toluene was added thereto, and the mixture was stirred, then allowed to stand still, and separated. The resulting organic layer was washed with 89 g (2.0% weight concentration) of an aqueous sodium hydroxide solution four times, and then further washed with 42 g (4.8% weight concentration) of an aqueous sodium chloride solution. After distilling off the solvent from the resulting organic layer under reduced pressure conditions, 27.0 g of a solution containing 21.2 g (93.5 mmol, yield of 100%) of Compound B was obtained. Here, the yield was quantitatively determined using HPLC in the following conditions.
¹H-NMR(CDCl₃)δ: 7.33-7.23(5H, m), 3.83-3.70(3H, m), 2.98(1H, d, J=10 Hz) 2.82(1H, dd, J=5 Hz ,10 Hz), 1.47(1H, d, J=8 Hz), 1.37(1H, dd, J=4Hz, 7 Hz), 1.29(3H, s), 0.99(3H, s)

### [HPLC Conditions]

Column: ZORBAX CN, 4.6 mmφ × 250 mm, 5 µm (manufactured by Agilent Technologies, Inc.)

Flow rate: 1.00 ml/min

Detection wavelength: UV 220 nm

Mobile phase: Solution A (hexane)/Solution B (tetrahydrofuran)

### Gradient conditions:

| | | | |
|---|---|---|---|
| Time (min.) | 0 | 5 | 30 |
| Solution A (%) | 95 | 95 | 75 |
| Solution B (%) | 5 | 5 | 25 |

Column temperature: 25°C

### Example 19

### [Steps A+B]: Synthesis of Compound B

Compound E (10.0 g) (46.45 mmol), 0.50 g (9.29 mmol) of sodium methoxide and 20 g of tetrahydrofuran were mixed and then cooled to 20°C. Thereto was added dropwise 10.73 g of a toluene solution (70% weight concentration, 37.15 mmol) of sodium bis(2-methoxyethoxy)aluminum hydride at 20°C over 2 hours, and the mixture was stirred at the same temperature for 1 hour. At this point, the conversion rate and the ratio of Compound A to over-reduced form were calculated in the same HPLC conditions as in Example 1. The reaction mixture was added dropwise to a mixed liquid of 1.52 g of sodium gluconate, 2.73 g of sodium cyanide and 20 g of water while maintaining a temperature of 4 to 7°C, and the mixture was stirred at the same temperature for 15 hours. Further, 14.4 g (12% weight concentration) of aqueous sodium hypochlorite was added dropwise at the same temperature, and 10 g of toluene was added thereto. Thereafter, the mixture was stirred, allowed to stand still, and separated. The resulting organic layer was washed with 20 g (10% weight concentration) of an aqueous sodium chloride solution at the same temperature once, and then further washed with 20 g of water twice. After distilling off the solvent from the resulting organic layer under reduced pressure conditions, a solution containing 10.3 g (yield of 97.6%) of Compound B was obtained. The yield was determined in the same HPLC conditions in Example 18.

Conversion rate from raw materials to product: 99.8%

Ratio of Compound A to over-reduced form: 99.3/0.7

### Example 20

### [Steps A+B]: Synthesis of Compound B

Compound E (10.0 g) (46.45 mmol), 0.50 g (9.29 mmol) of sodium methoxide and 20 g of toluene were mixed and then cooled to 10°C. Thereto was added dropwise 10.73 g of a toluene solution (70% weight concentration, 37.15 mmol) of sodium bis (2-methoxyethoxy) aluminum hydride at 10°C over 2 hours, and the mixture was stirred at the same temperature for 16 hours. At this point, the conversion rate and the ratio of Compound A to over-reduced form were calculated in the same HPLC conditions as in Example 1. The reaction mixture was added dropwise to a mixed liquid of 1.52 g of sodium gluconate, 2.73 g of sodium cyanide, 1.39 g of acetic acid and 20 g of water while maintaining a temperature of 4 to 8°C, and the mixture was stirred at the same temperature for 24 hours. Further, 14.4 g (12% weight concentration) of aqueous sodium hypochlorite was added dropwise at the same temperature, and 10 g of toluene was added thereto. Thereafter, the mixture was stirred, allowed to stand still, and separated. The resulting organic layer was washed with 20 g (10% weight concentration) of an aqueous sodium chloride solution at the same temperature once, and further washed with 20 g of water twice. After distilling off the solvent from the resulting organic layer under reduced pressure conditions, a solution containing 9.90 g (yield of 94.3%) of Compound B was obtained. The yield was determined in the same HPLC conditions in Example 18.

Conversion rate from raw materials to product: 99.8%

Ratio of Compound A to over-reduced form: 99.2/0.8

### Example 21

### [Steps A+B]: Synthesis of Compound B

Compound E (10.0 g) (46.45 mmol), 0.50 g (9.29 mmol) of sodium methoxide and 20 g of toluene were mixed and then cooled to 10°C. Thereto was added dropwise 10.73 g of a toluene solution (70% weight concentration, 37.15 mmol) of sodium bis (2-methoxyethoxy) aluminum hydride at 10°C over 2 hours, and the mixture was stirred at the same temperature for 2 hours. At this point, the conversion rate and the ratio of Compound A to over-reduced form were calculated in the same HPLC conditions as in Example 1. Thereafter, the reaction mixture liquid was added dropwise to a mixed liquid of 1.52 g of sodium gluconate, 2.73 g of sodium cyanide, 5.0 g of isopropyl alcohol and 20 g of water while maintaining a temperature of 5 to 7°C, and the mixture was stirred at the same temperature for 18 hours. To the resulting reaction mixture liquid was added dropwise 14.4 g (12% weight concentration) of aqueous sodium hypochlorite at the same temperature, and was further added 10 g of toluene, and then the mixture was stirred. Thereafter, 14.6 g (10% weight concentration) of an aqueous sodium sulfite solution was added dropwise at the same temperature, and the mixture was stirred, then allowed to stand still, and separated. The resulting organic layer was washed with 20 g (10% weight concentration) of an aqueous sodium chloride solution at the same temperature once, and further washed with 20 g of water twice. After distilling off the solvent from the resulting organic layer under reduced pressure conditions, a solution containing 10.00 g (yield of 96.0%) of Compound B was obtained. The yield was determined in the same HPLC conditions in Example 18.

Conversion rate from raw materials to product: 99.8%

Ratio of Compound A to over-reduced form: 99.3/0.7

### Example 22

### [Steps A+B]: Synthesis of Compound B

Compound E (14.0 g) (65.0 mmol), 1.46 g (13.0 mmol) of potassium tert-butoxide and 28.0 g of tetrahydrofuran were mixed and then cooled to -10°C. Thereto was added dropwise 15. 0 g of a toluene solution (70% weight concentration, 52.0 mmol) of sodium bis(2-methoxyethoxy)aluminum hydride at -10°C over 2 hours, and the mixture was stirred at the same temperature for 20 hours. The reaction mixture was added dropwise to 2.42 g (48.3% weight concentration) of a methanol solution of acetic acid. To this reaction liquid was added dropwise 17.8 g (21.5% weight concentration, 78.2 mmol) of an aqueous sodium cyanide solution at 5°C over 35 minutes, and the mixture was stirred at the same temperature for 11 hours. Thereafter, 0.78 g of acetic acid was added dropwise thereto at 5°C over 5 minutes, and the mixture was stirred at the same temperature for 15 hours. Further, at the same temperature, 0.32 (6.52 mmol) of sodium cyanide was added, and the mixture was stirred for 3 hours, furthermore, 0.64 g (13.1 mmol) of sodium cyanide was added at the same temperature, and the mixture was stirred for 3 hours. The resulting reaction mixture liquid was added dropwise to a mixed liquid of 4.44 g of sodium gluconate and 49.0 g (2.85% weight concentration) of an aqueous sodium hydroxide solution at 10°C, and the mixture was stirred at the same temperature, then allowed to stand still and separated. The resulting
organic layer was washed with 62.5 g (2.0% weight concentration) of an aqueous sodium hydroxide solution three times, and then further washed with 29.4 g (4.8% weight concentration) of an aqueous sodium chloride solution. After distilling off the solvent from the resulting organic layer under reduced pressure conditions, 17.8 g of a solution containing 13.44 g (59.4 mmol, yield of 91%) of Compound B was obtained. The yield was quantitatively determined using GC in the following conditions.

### [GC Conditions]

Column: DB-1, 0.25 mmφ × 30 m, 0.25 µm (J&W Scientific, Inc.)

Detection method: FID

Flow rate: 1 ml/min (helium)

### Column temperature:

| | | | | |
|---|---|---|---|---|
| Time (min.) | 0 | 10 | 46 | 55 |
| Temperature (°C) | 100 | 100 | 280 | 280 |

Injector temperature: 150°C

Detector temperature: 280°C

### Example 23

### [Step B]: Synthesis of Hydrochloride salt of Compound B

To 8.83 g of a concentrate containing 7.38 g (32.6 mmol) of Compound B were added 20 ml of ethyl acetate and 20 ml of methyl ethyl ketone, and the mixture was cooled to 0°C. Thereafter, thereto was added dropwise 9 ml of a 4 N HCl ethyl acetate solution, the mixture was stirred at 0°C for 25 minutes, and then the precipitated crystals were collected by filtration. The resulting crystals were washed with 15 ml of methyl ethyl ketone and dried under reduced pressure to obtain 7.60 g of white crystals containing 7.60 g (28.9 mmol, yield of 88.7%) of hydrochloride salt of Compound B. The yield was quantitatively determined using HPLC in the same HPLC conditions in Example 18.
¹H-NMR(D₂O)δ: 8.57(1h, s), 7.55-7.12(5H, m), 5.05(2H, s), 4.24(1H, m), 4.02(1H, d, J=16 Hz), 2.73(1H, br.s), 2.49(1H, br.s), 1.27(3H, s), 0.88(3H, s).

### Example 24

### [Step C]: Synthesis of methyl (1R,2S,5S)-3-benzyl-6,6-dimethyl-3-azabicyclo[3.1.0]hexane-2-carboxylate (Compound C1)

Compound B (15.0 g) (66.3 mmol) and 23.8 g of methanol were mixed, and cooled to -30°C, then 37.2 g (1.02 mol) of hydrogen chloride gas was blown thereinto at -10°C or lower over 2 hours and 30 minutes, and the mixture was stirred at a temperature of -15°C to -10°C for 2 hours. Thereafter, the resulting reaction mixture liquid was added dropwise to 300 g of water at 20°C or lower. After the completion of dropwise addition, while stirring at 20°C, the mixture was adjusted to have a pH of 9 with 70.4 g (0.51 mol) of potassium carbonate, and extracted with 100 ml of toluene twice. The resulting organic layers were combined and mixed, and washed with 5% saline. The resulting organic layer was dried over anhydrous magnesium sulfate and filtered. The resulting filtrate was concentrated under reduced pressure to distill off the solvent, obtaining 16.0 g of a solution containing 14.4 g (55.5 mmol: yield of 83.7%) of Compound C1. The yield was quantitatively determined using GC in the same GC conditions in Example 22. ¹H-NMR(CDCl₃)δ: 7.30-7.19(5H, m), 3.73(2H, d, J=16 Hz), 3.70(3H, s), 3.52(1H, s), 3.15(1H, dd, J=9 Hz, 5 Hz), 2.69(1H, d, J=9 Hz), 1.37-1.30(2H, m), 1.17(3H, s), 1.00(3H, s)

### Example 25

### [Step C]: Synthesis of Compound C1

Hydrochloride salt of Compound B (2.63 g) (10.0 mmol), 0.48 g of methanol and 15.8 g of toluene were mixed, and cooled to -10°C, then hydrogen chloride gas was blown thereinto while maintaining the temperature at -10°C or lower over 2 hours. This mixed liquid was stirred at -10°C for 18.5 hours, and then heated to 25°C. Thereafter, 1.78 g (15.0 mmol) of thionyl chloride and 3.20 g of a methanol solution were added dropwise thereto in turn at a temperature of 25 to 30°C. After the completion of dropwise addition, the reaction mixture was heated to 66°C, and stirred at the same temperature for 9.5 hours. Thereafter, 1.19 g (10.0 mmol) of thionyl chloride was further added thereto, and the mixture was stirred at 66°C for 2 hours. This reaction mixture liquid was cooled to room temperature, 32 g of an aqueous sodium carbonate solution (7% weight concentration) was added thereto, and the mixture was stirred, then allowed to stand still, and separated. The resulting organic layer was washed with aqueous sodium chloride (5% weight concentration), to obtain an organic layer containing 2.07 g (8.0 mmol: yield of 80.0%) of Compound C1. The yield was quantitatively determined using HPLC in the same HPLC conditions as in Example 1.

### Example 26

### [Step C]: Synthesis of Compound C1

To 10.2 g of a toluene solution containing 4.32 g (19.1 mmol) of Compound B were added 21.8 g of toluene and 0.83 g of methanol, and the mixture was cooled to -10°C. To this mixed liquid was added dropwise 1.92 g (20.0 mmol) of methanesulfonic acid at -10°C over 15 minutes, and then 6.5 g (178.3 mmol) of hydrogen chloride gas was blown thereinto while maintaining the temperature at -105°C or lower. The reaction mixture was stirred at -10°C for 1.5 hours, and then stirred at 5°C for 20 hours. Thereafter, the mixture was heated to 20°C, and 9.61 g of methanol was added dropwise thereto at a temperature of 20 to 30°C, then 7.69 g (80.0 mmol) of methanesulfonic acid was added dropwise thereto. After the completion of dropwise addition, the reaction mixture liquid was heated to 60°C, and stirred at the same temperature for 23.5 hours. Thereafter, the mixture was cooled to 25°C, 30 g of water was added thereto, and the mixture was adjusted to have a pH of 7.6 with 6.45 g (25% weight concentration) of aqueous ammonia. This mixed solution was filtered, then allowed to stand still, and separated. In the resulting organic layer, 4.05 g (15.6 mmol: yield of 81.7%) of Compound C1 was contained. The yield was quantitatively determined using HPLC in the same HPLC conditions as in Example 1.

### Example 27

### [Step C]: Synthesis of (1R,2S,5S)-3-benzyl-6,6-dimethyl-3-azabicyclo[3.1.0]hexane-2-carboxamide (Compound C2)

A toluene solution (55.8 g) containing 12.0 g (53.0 mmol) of Compound B was added dropwise to 36.8 g (45% weight concentration) of hydrochloric acid at 4 to 7°C over 2 hours, and the mixture was stirred at the same temperature for 23 hours. To the reaction mixture liquid was added 19.4 g of water, then 35.8 g of an aqueous sodium hydroxide solution (48% weight concentration) was added dropwise thereto, and the pH was adjusted to 10. This mixed liquid was stirred, then allowed to stand still, and separated. To the resulting aqueous layer was added 26.6 g of an aqueous sodium chloride solution (5% weight concentration) for washing. After distilling off the solvent from the resulting organic layer under reduced pressure conditions, a solution containing 11.9 g (48.7 mmol, yield of 91.9%) of Compound C2 was obtained. The yield was quantitatively determined using HPLC in the same conditions as in Example 1.
¹H-NMR(CDCl₃)δ: 7.36-7.24(6H, m), 5.58(1H, br.s), 3.79(1H, d, J=13 Hz), 3.58(1H, d, J=13 Hz), 3.34(1H, dd, J=10 Hz, 6 Hz), 3.19(1H d, J=2 Hz), 2.38(1H, dd, J=10 Hz, 2 Hz), 1.49(1H dd, J=8 Hz, 2 Hz), 1.34-1.39(1H, m), 1.04(3H, s), 0.96(3H, s).

### Example 28

### [Step C]: Synthesis of Compound C2

A toluene solution (53.7 g) containing 37.6g (166.2 mmol) of Compound B was added dropwise to 173.3 g of hydrochloric acid (35% weight concentration) at 17°C over 20 minutes, and the mixture was stirred at the same temperature for 48 hours. To the reaction mixture were added 76 g of water and 115 g of toluene, then 142 g of an aqueous sodium hydroxide solution (48% weight concentration) was added dropwise thereto, and the pH was adjusted to 9. The mixture was allowed to stand still and separated, 78 g of toluene was added to the resulting aqueous layer, and the mixture was stirred, allowed to stand still and separated. All of the resulting organic layers were mixed, and then thereto was added 84 g (5% weight concentration) of an aqueous sodium chloride solution for washing. After distilling off the solvent from the resulting organic layer under reduced pressure conditions, a solution containing 34.6 g (141.6 mmol, yield of 85.2%) of Compound C2 was obtained. The yield was quantitatively determined using HPLC in the same HPLC conditions as in Example 1.

### Example 29

### [Step C]: Synthesis of Compound C2

Hydrochloric acid (23.1 g) (35% weight concentration) and 5.90 g of a toluene solution containing 5.04 g (22.3 mmol) of Compound B were mixed, and the mixture was stirred at 15°C for 4 hours. Thereafter, 10. 9 g of hydrogen chloride gas was blown thereinto, and the mixture was stirred for further 2 hours. To the reaction mixture were added dropwise 16 g of toluene and 67 g of an aqueous sodium hydroxide solution (30% weight concentration), and the pH was adjusted to 7. The mixture was allowed to stand still, and the separated aqueous layer was extracted with 10 g of toluene. The resulting organic layers were mixed to obtain a solution containing 4.42 g (18.1 mmol, yield of 88.6%) of Compound C2. The yield was quantitatively determined using HPLC in the same HPLC conditions as in Example 1.

### Example 30

### [Step C]: Synthesis of Compound C2

A toluene solution (81.7 g) containing 10. 0 g (44.2 mmol) of Compound B, and 2.12 g (66.3 mmol) of methanol were mixed, and cooled to 0°C, then hydrogen chloride gas was blown thereinto at a temperature of 0 to 6°C for 1 hour, and the mixture was heated to 10°C. The mixture was stirred at the same temperature for 3 hours, then hydrogen chloride gas was blown thereinto at 10°C for 10 minutes, and the mixture was stirred for 1 hour. Further, hydrogen chloride gas was blown thereinto at 10°C for 5 minutes, and the mixture was stirred for 1 hour. Moreover, hydrogen chloride gas was blown thereinto at 10°C for 5 minutes, and the mixture was stirred for 1 hour. Furthermore, hydrogen chloride gas was blown thereinto at 10°C for 5 minutes, and the mixture was stirred for 15 hours. To the reaction mixture were added dropwise 29 g of an aqueous sodium hydroxide solution (5% weight concentration) and 35 g of an aqueous sodium hydroxide solution (15% weight concentration), and the pH was adjusted to 12. This mixed liquid was stirred, then allowed to stand still, and separated, to obtain a toluene layer. To the aqueous layer was added 50 g of toluene, and the mixture was extracted. The resulting toluene layers were combined and dried over sodium sulfate to obtain a solution containing 9.14 g (37.4 mmol, yield of 84.6%) of Compound C2. The yield was quantitatively determined using HPLC in the same HPLC conditions as in Example 1.

### Example 31

### [Step C]: Synthesis of Hydrochloride salt of Compound C1

To 32 g of methanol cooled to 5°C was added dropwise 21.4 g (25% weight concentration, 66.9 mmol) of fuming sulfuric acid at a temperature of 5 to 15°C over 1.5 hours, then the mixture was heated to 74°C. The mixture was stirred at the same temperature for 0.5 hours, then 15.3 g of a toluene solution containing 8.0 g (32.7 mmol) of Compound C2 was added dropwise thereto at 74°C over 0.5 hours, and the mixture was stirred at the same temperature for 10 hours. The reaction mixture was cooled to 25°C, 16 g of toluene was added thereto, and the mixture was stirred. This toluene mixed liquid and 19.9 g (28% weight concentration) of aqueous ammonia were added dropwise at the same time to 17.6 g of water at 5°C while maintaining a pH of 9 to 11. After the completion of dropwise addition, to the mixed liquid was added 16 g of water, and the mixture was stirred, then allowed to stand still and separated to obtain a toluene layer. The aqueous layer was extracted with 16 g of toluene. The resulting toluene layers were combined and washed with 16 g of water, and the solvent was distilled off under reduced pressure conditions to obtain 9.75 g of a concentrate containing 8.09 g (31.2 mmol, yield of 95.2%) of Compound C1. The yield was quantitatively determined using HPLC in the same HPLC conditions as in Example 1.

To this concentrate were added dropwise 55 g of toluene and 4.8 g (28% weight concentration) of a hydrogen chloride 2-propanol solution at 5°C, the mixture was stirred at the same temperature for 1 hour, and then the precipitated crystals were collected by filtration. The resulting crystals were washed with a mixed solution of 8 g of toluene and 0.8 g of 2-propanol, then further with 16 g of toluene, and dried under reduced pressure to obtain 9.02 g of white crystals containing 8.28 g (28.0 mmol, yield of 85.5%) of hydrochloride salt of Compound C1. The yield was quantitatively determined using HPLC in the same HPLC conditions as in Example 1.

### Example 32

### [Step C]: Synthesis of Hydrochloride salt of Compound C1

A toluene solution (53.1 g) containing 34.7 g (142 mmol) of Compound C, and 69.5 g of methanol were mixed, and 71.2 g (98% weight concentration, 711 mmol) of concentrated sulfuric acid was added dropwise thereto at a temperature of 25 to 45°C over 30 minutes. The reaction mixture was heated to 60 to 65°C, and stirred at the same temperature for 40 hours, then cooled to room temperature. Thereafter, 70 g of toluene was added thereto, and the mixture was stirred, then this toluene mixed liquid and 50.2 g (28% weight concentration) of aqueous ammonia were added dropwise at the same time to 76 g of water while maintaining a pH of 9 to 11. After the completion of dropwise addition, the mixed liquid was allowed to stand still and separated, and the resulting organic layer was washed with 70 g of water. The solvent was distilled off from this organic layer under reduced pressure conditions, to obtain 52.7 g of a concentrate containing 32.6 g (125.6 mmol: yield of 88.4%) of Compound C1. The yield was quantitatively determined using HPLC in the same HPLC conditions as in Example 1.

To this concentrate was added 208 g of toluene, added dropwise 23.1 g (25% weight concentration) of a hydrogen chloride 2-propanol solution at 7 to 12°C, and the mixture was stirred at the same temperature for 1 hour, then the precipitated crystals were collected by filtration. The resulting crystals were washed with a mixed solution of 35 g of toluene and 4 g of 2-propanol, then further with 70 g of toluene, and dried under reduced pressure to obtain 36.6 g of white crystals containing 35.2 g (118.8 mmol, yield of 83.6%) of hydrochloride salt of Compound C2. The yield was quantitatively determined using HPLC in the same HPLC conditions as in Example 1.

### Example 33

### [Step C]: Synthesis of Compound C1

A toluene solution (0.44 g) containing 0.37 g (1. 51 mmol) of Compound C2, 2.6 g of toluene and 0.77 g of methanol were mixed, and 0.58 g (7.49 mmol) of methanesulfonic acid was added dropwise thereto at a temperature of 25 to 50°C over about 5 minutes. The reaction mixture liquid was heated to 66°C, and stirred at the same temperature for 24 hours, then cooled to room temperature. In the reaction mixture solution, 0.38 g (1.47 mmol: yield of 97.5%) of Compound C1 was contained. The yield was quantitatively determined using HPLC in the same HPLC conditions as in Example 1.

### Example 34

### [Step D]: Synthesis of methyl (1R,2S,5S)-6,6-dimethyl-3-azabicyclo[3.1.0]hexane-2-carboxylate hydrochloride salt (Compound D)

Compound C1 (3.23 g) (12.5 mmol), 1.43 g (13.7 mmol) of 35% hydrochloric acid, 0.59 g of 10% Pd/C (50% hydrous type) and 22.7 g of methanol were mixed, and heated to 45°C. The inside of the reaction vessel was replaced with hydrogen gas, and the mixture was stirred at the same temperature for 5 hours. The reaction vessel was opened, then toluene was added thereto, and the solvent was distilled off under reduced pressure conditions, to obtain 3.23 g of a solid containing 2.28 g (11.1 mmol: yield of 88.8%) of Compound D. The yield was quantitatively determined using HPLC in the following conditions.

### [HPLC Conditions]

Column: Waters Xbridge C18, 4.6 mmφ × 150 mm, 3.5 µm (manufactured by Waters Corporation)

Flow rate: 1.0 ml/min

Detection wavelength: UV 210 nm

### Mobile phase:

Solution A (10 mM Na₂HPO₄/acetonitrile = 95/5)
Solution B (acetonitrile/water = 80/20)

### Gradient conditions:

| | | |
|---|---|---|
| Time (min.) | 0 | 30 |
| Solution A (%) | 100 | 10 |
| Solution B (%) | 0 | 90 |

Column temperature: 40°C

### Example 35

### [Step D]: Synthesis of Compound D

Hydrochloride salt of Compound C1 (12.0 g) (content of 96.1%, 39.0 mmol), 10% Pd/C (50% hydrous type) and 2-propanol were mixed, and the inside of the reaction vessel was replaced with nitrogen, then pressurized to 0.40 MPa with hydrogen gas, and the mixture was stirred at 25°C for 8 hours. The inside of the reaction vessel was decompressed and then heated to 50°C, a glass filter was precoated with 0.60 g of a filter aid, and the reaction mixture liquid was filtered. Further, the filtration residue was washed with 2-propanol at 50°C, and the resulting washing liquid was mixed with the filtrate. In this mixture (40.0 g), 7.85g (38. 2 mmol: yield of 97.9%) of Compound D was contained. The yield was quantitatively determined using HPLC in the same HPLC conditions in Example 34.

After 40.0 g of this mixed liquid was concentrated to 22.1 g, 5.6 g of 2-propanol was added thereto, and the mixture was heated to 40°C. Thereto was added 0.01 g of a seed crystal of Compound D, and the mixture was stirred for 1 hour. Thereafter, 39.6 g of methyl t-butyl ether was added dropwise thereto at the same temperature over 1.5 hours, and then the mixture was stirred for 1 hour and cooled to -5°C. The mixture was stirred at -5°C for 17 hours, and then the precipitated crystals were collected by filtration. The resulting crystals were washed with a mixed liquid of 11.5 g of 2-propanol and 5.8 g of methyl t-butyl ether at -5°C, then further with 8.6 g of methyl t-butyl ether at -5°C, and dried under reduced pressure to obtain white crystals containing 7.1 g (content of 100%, 34.6 mmol: yield of 88.6%) of Compound D. The yield was quantitatively determined using HPLC in the same HPLC conditions in Example 34.

### Example 36

### Synthesis of (1R,5S)-3-benzyl-6,6-dimethyl-3-azabicyclo[3.1.0]hexan-2-one (Compound E)

A 200-mL autoclave was charged with 19.3 g of a toluene solution containing 15.0 g (118.6 mmol) of (1R,5S)-6,6-dimethyl-3-oxabicyclo[3.1.0]hexan-2-one (Compound F), 25.7 g (239.7 mmol) of benzylamine and 64.8 g (3.6 mmol) of water, and the resultant was mixed and then heated to 180°C under a pressure condition of 0.9 MPa. The mixture was stirred at the same temperature for 10 hours and cooled to 35°C, then, 12.2 g (117.1 mmol) of 1 N hydrochloric acid was added dropwise to the reaction mixture, and the pH was adjusted to 4. The reaction mixture liquid was extracted with 45 g of methyl tert-butyl ether twice. As a result of quantitative determination of the reaction mass, 24.1 g (111.8 mmol, yield of 94.3%) of Compound E was contained. The resulting organic layers were mixed, and washed with 45 g of water once, and the organic layer was concentrated under reduced pressure conditions to distill off the solvent. To this concentrate was added 64 g of heptane, and the mixture was heated to 65°C, then 1.5 g of activated carbon was added thereto, and the mixture was stirred at the same temperature for 0.5 hours. This mixture was filtered, and the filtration residue was washed with 45 g of heptane. The resulting filtrate and the washing liquids were mixed and concentrated under reduced pressure conditions to distill off the solvent. To the resulting concentrate was added 60 g of heptane, and the mixture was heated to 47°C. Thereto was added 0.02 g of (1R,5S)-3-benzyl-6,6-dimethyl-3-azabicyclo[3.1.0]hexan-2-one, and the mixture was stirred for 1 hour. Thereafter, the mixture was cooled to 5°C, and the precipitated crystals were collected by filtration. The resulting crystals were further washed with 38 g of heptane at 5°C and then dried under reduced pressure to obtain 21.57 g of white crystals containing 21.52 g (99.9 mmol, yield of 84.3%) of Compound E. The yield was quantitatively determined using HPLC in the same HPLC conditions as in Example 1. ¹H-NMR(CDCl₃)δ: 7.34-7.24(5H, m), 4.50(1H, d, J=14 Hz), 4.15(1H, d, J=15 Hz), 3.37(1H, dd, J=7 Hz, 11 Hz), 2.99(1H, d, J=11 Hz), 1.85(1H, d, J=7 Hz), 1.58(1H, dd, J=7 Hz, 7 Hz), 1.09(3H, s), 0.94(3H, s)

### Example 37

### Synthesis of Compound E

A 200-mL autoclave was charged with 9.82 g of a toluene solution containing 7.58 g (60.1 mmol) of Compound F, 12.87 g (120 mmol) of benzylamine and 32.5 g (1.80 mol) of water, and the resultant was mixed and then heated to 170°C under a pressure condition of 0.7 MPa. The mixture was stirred at the same temperature for 15 hours to obtain a reaction mixture liquid containing 12.3 g (57.3 mmol, yield of 95.6%) of Compound E. The yield was quantitatively determined using HPLC in the same HPLC conditions as in Example 1.

### Examples 38 to 43

### Synthesis of Compound E

The amount of water described in Example 37 was changed to the amount described in Table 1, and the reaction was carried out in the same manner as in Example 37 except for the amount of water. The results are shown in Table 1.

**[Table 1]**

| | Example 38 | Example 39 | Example 40 | Example 41 | Example 42 | Example 43 |
|---|---|---|---|---|---|---|
| Compound F | 11.34 g | 15.12 g | 7.58 g | 7.57 g | 3.79 g | 1.89 g |
| | 89.9 mmol | 119.9 mmol | 60.1 mmol | 60.0 mmol | 30.0 mmol | 15.0 mmol |
| Amount of water | 1.62 g | 6.48 g | 10.90 g | 21.61 g | 54.01 g | 56.80 g |
| | 89.9 mmol | 359.6 mmol, | 0.61 mol | 1.20 mol | 3.00 mol | 3.16 mol |
| Yield | 52.70% | 87.30% | 92.60% | 92.90% | 94.90% | 90.50% |

### Example 44

### Synthesis of Compound E

A 200-mL autoclave was charged with 14.71 g of a toluene solution containing 11.36 g (90.1 mmol) of Compound F, 19.3 g (180 mmol) of benzylamine, 16.2 g (899 mol) of water and 22.7 g of methanol, and the resultant was mixed and then heated to 160°C under a pressure condition of 1.0 MPa. The mixture was stirred at the same temperature for 21 hours to obtain a reaction mixture containing 17.5 g (81.3 mmol, yield of 90.3%) of Compound E. The yield was quantitatively determined using HPLC in the same HPLC conditions as in Example 1.

### Example 45

### Synthesis of Compound E

A 200-mL autoclave was charged with 5.05 g (39.6 mmol, content of 99.0%) of Compound F, 8.49 g (79.3 mmol) of benzylamine, 17.96 g of water and 10.03 g of methanol, and the resultant was mixed and then heated to 165°C under a pressure condition of 0.9 MPa. The mixture was stirred at the same temperature for 12 hours to obtain a reaction mixture containing 8.39 g (39.0 mmol, yield of 98.5%) of Compound E.

Thereafter, 3.99 g of 35% hydrochloric acid was added thereto to adjust the pH to 5, and then the mixture was extracted with 15 g of toluene twice. The resulting organic layers were combined and concentrated under reduced pressure conditions to distill off the solvent, and then 23 g of heptane was flown thereinto. The mixture was cooled to 10°C, and the precipitated crystals were collected by filtration, washed with 14 g of heptane, and dried, to obtain 7.05 g (32.7 mmol, yield of 82.5%) of Compound E. The yield was quantitatively determined using HPLC in the same HPLC conditions as in Example 1.

### Example 46

### Synthesis of Compound E

A 200-mL autoclave was charged with 14.69 g of a toluene solution containing 11.34 g (90.0 mmol) of Compound F, 16.39 g (153 mmol) of benzylamine, 16.2 g (900 mol) of water and 22.78 g of methanol, and the resultant was mixed and then heated to 170°C under a pressure condition of 1.2 MPa. The mixture was stirred at the same temperature for 15 hours to obtain a reaction mixture containing 17.96 g (83.4 mmol, yield of 92.8%) of Compound E. The yield was quantitatively determined using HPLC in the same HPLC conditions as in Example 1.

### Example 47

### Synthesis of Compound E

A 200-mL autoclave was charged with 9.80 g of a toluene solution containing 7.57 g (60.0 mmol) of Compound F, 32.1 g (300 mmol) of benzylamine and 10.8 g (600 mol) of water, and the resultant was mixed and then heated to 170°C under a pressure condition of 0.6 MPa. The mixture was stirred at the same temperature for 15 hours to obtain a reaction mixture solution containing 11.8 g (54.9 mmol, yield of 91.5%) of Compound E. The yield was quantitatively determined using HPLC in the same HPLC conditions as in Example 1.

### Example 48

### Synthesis of Compound E

A 200-mL autoclave was charged with 14.69 g of a toluene solution containing 11.34 g (89.9 mmol) of Compound F, 19.26 g (179.8 mmol) of benzylamine, 16.25 g of water, 22.71 g of methanol and 3.51 g (18.0 mmol) of p-toluenesulfonic acid monohydrate, and the resultant was mixed and then heated to 170°C under a pressure condition of 1.2 MPa. The mixture was stirred at the same temperature for 15 hours, and the resulting reaction mixture liquid was quantitatively determined. The yield of Compound E based on Compound F was 95.4%. The yield was quantitatively determined using HPLC in the same HPLC conditions as in Example 1.

### Example 49

### Synthesis of Compound E

A 100-mL autoclave was charged with 4.90 g of a toluene solution containing 3.78 g (30.0 mmol) of Compound F, 3.54 g (33.0 mmol) of benzylamine, 5.5 g (305 mmol) of water and 0.38 g (3.00 mmol) of γ-alumina, and the resultant was mixed and then heated to 170°C in the autoclave. The mixture was stirred at the same temperature for 15 hours, and water was added to the resulting reaction mixture liquid. The mixture was stirred and then separated, and the organic layer and the water layer were quantitatively determined. The yield of Compound E based on Compound F was 89.9%. The yield was quantitatively determined using HPLC in the same HPLC conditions as in Example 1.

### INDUSTRIAL APPLICABILITY

According to the present invention, a proline compound represented by formula (1) or a salt thereof useful as an intermediate for the production of a medicinal or agrochemical agent or the like can be produced in a high yield.

## Claims

1. A method for producing a proline compound represented by formula (1), 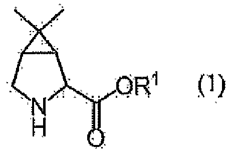 wherein R¹ represents an optionally substituted aliphatic hydrocarbon group or a hydrogen atom,
or a salt thereof, comprising the following steps A to D:
[Step A]:
a step of reacting a pyrrolidinone compound represented by formula (2), 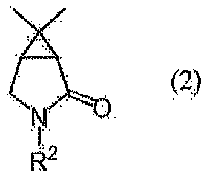 wherein R² represents a group represented by C(R¹⁰)₂Ar, Ar is an optionally substituted phenyl group or an optionally substituted naphthyl group, and two R^{10†}s are each independently a hydrogen atom, an optionally substituted alkyl group or an optionally substituted phenyl group,
with a reducing agent represented by formula (3),
**(M^{s+})_{1/s}H_{4-mA}I-(OR³)ₘ**
wherein R³ represents an optionally substituted alkyl group,
M^{s+} represents a metal ion, s represents a valence of the metal ion, and m represents 1, 2 or 3,
in the presence of a base to obtain a pyrrolidinol compound represented by formula (4) or a salt thereof;
[Step B]:
a step of reacting the pyrrolidinol compound represented by formula (4), 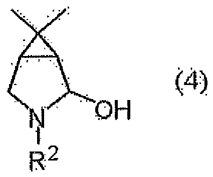
wherein R² represents the same meaning as described above, or the salt thereof with a cyanating agent to obtain a cyanopyrrolidine compound represented by formula (5) or a salt thereof;
[Step C]:
a step of hydrolyzing or alcoholyzing the cyanopyrrolidine compound represented by formula (5), 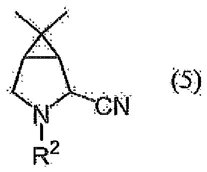 wherein R² represents the same meaning as described above, or the salt thereof to obtain a protected proline compound represented by formula (6) or a salt thereof; and
[Step D]:
a step of substituting R² in the protected proline compound represented by formula (6), 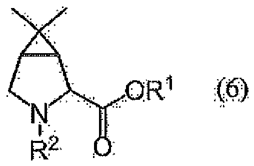 wherein R¹ and R² each represent the same meanings as described above,
or the salt thereof by a hydrogen atom.

2. The production method according to claim 1, comprising producing the pyrrolidinone compound represented by formula (2) by the following step E, and subsequently carrying out the steps A to D:
[Step E]:
a step of reacting a compound represented by formula (8) 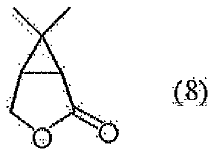 with a compound represented by formula (9),
**H₂N-R²**
wherein R² represents the same meaning as described above.

3. The production method according to claim 1 or 2, wherein the reducing agent represented by formula (3) in the step A is a compound represented by a formula,
**(M^{s+})_{1/s}H₄₋ₘAI⁻[O(CH₂)ₙOR⁴]ₘ**
wherein R⁴ represents an alkyl group or a cycloalkyl group, n represents 1, 2, 3 or 4, M^{s+}, and s and m each represent the same meaning as described above.

4. The production method according to claim 3, wherein the reducing agent represented by formula (3) in the step A is sodium bis(2-methoxyethoxy)aluminum hydride.

5. The production method according to any of claims 1 to 4, wherein the base in the step A is an alkali metal alkoxide.

6. The production method according to any of claims 1 to 4, wherein the base in the step A is N,N'-dimethylethylenediamine.

7. The production method according to any of claims 1 to 6, wherein the cyanating agent in the step B is a metal cyanide or hydrogen cyanide.

8. The production method according to any of claims 1 to 7, wherein the step C is carried out through an imidate compound represented by formula (7), 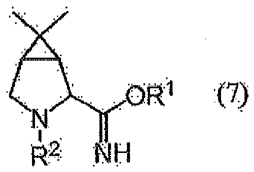 wherein R¹ and R² each represent the same meanings as described above,
or a tautomer thereof.

9. The production method according to any of claims 1 to 8, wherein the proline compound represented by formula (1) or the salt thereof is a proline compound represented by formula (1b), 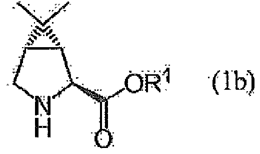 wherein R¹ represents the same meaning as described above, or a salt thereof.

10. A pyrrolidinone compound represented by formula (2), 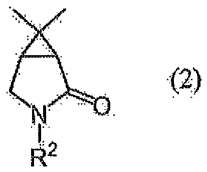 wherein R² represents a group represented by C(R¹⁰)₂Ar, Ar is an optionally substituted phenyl group or an optionally substituted naphthyl group, and two R¹⁰'s are each independently a hydrogen atom, an optionally substituted alkyl group or an optionally substituted phenyl group.

11. A pyrrolidinol compound represented by formula (4), 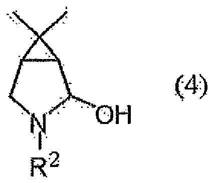 wherein R² represents a group represented by C(R¹⁰)₂Ar, Ar is an optionally substituted phenyl group or an optionally substituted naphthyl group, and two R¹⁰'s are each independently a hydrogen atom, an optionally substituted alkyl group or an optionally substituted phenyl group,
or a salt thereof.

12. A cyanopyrrolidine compound represented by formula (5), 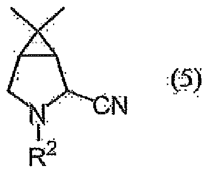 wherein R² represents a group represented by C(R¹⁰)₂Ar, Ar is an optionally substituted phenyl group or an optionally substituted naphthyl group, and two R¹⁰'s are each independently a hydrogen atom, an optionally substituted alkyl group or an optionally substituted phenyl group,
or a salt thereof.

13. An imidate compound represented by formula (7), 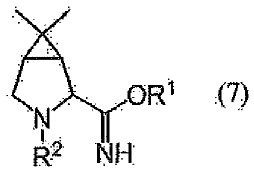 wherein R¹ represents an optionally substituted aliphatic hydrocarbon group or a hydrogen atom, R² represents a group represented by C (R¹⁰)₂Ar, Ar is an optionally substituted phenyl group or an optionally substituted naphthyl group, and two R¹⁰'s are each independently a hydrogen atom, an optionally substituted alkyl group or an optionally substituted phenyl group,
or a tautomer thereof.

14. A protected proline compound represented by formula (6), 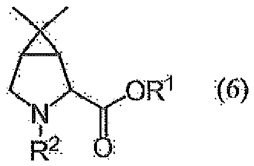 wherein R¹ represents an optionally substituted aliphatic hydrocarbon group or a hydrogen atom, R² represents a group represented by C (R¹⁰)₂Ar, Ar is an optionally substituted phenyl group or an optionally substituted naphthyl group, and two R¹⁰'s are each independently a hydrogen atom, an optionally substituted alkyl group or an optionally substituted phenyl group,
or a salt thereof.
